# EUROPEAN PATENT SPECIFICATION

(11) **EP 1 940 463 B1**
(45) Date of publication and mention of the grant of the patent: **29.03.2017**
(21) Application number: 06825089.3
(22) Date of filing: 22.09.2006
(51) Int. Cl.: A61K 39/385, A61K 38/19, C07K 14/525

(54) **METHOD FOR ENHANCING IMMUNE RESPONSES IN MAMMALS**
VERFAHREN ZUR VERSTÄRKUNG DER IMMUNANTWORTEN BEI SÄUGETIEREN
PROCÉDÉ VISANT À RENFORCER DES RÉPONSES IMMUNES CHEZ DES MAMMIFÈRES

(30) Priority: 22.09.2005 US 234057
(43) Date of publication of application: 09.07.2008
(73) Proprietor: Cytologic, Inc., Fort Collins CO 80526 (US)
(72) Inventor: HOWELL, Mark Douglas, Fort Collins, CO 80526 (US)
(74) Representative: Leissler-Gerstl, Gabriele
(86) International application number: PCT/US2006/037171
(87) International publication number: WO 2007/038386

(56) References cited:
- EP-A2- 0 247 860
- WO-A2-01/43770
- US-B1- 6 379 708
- SELINSKY C L ET AL: "MULTIFACETED INHIBITION OF ANTI-TUMOUR IMMUNE MECHANISMS BY SOLUBLE TUMOUR NECROSIS FACTOR RECEPTOR TYPE I", IMMUNOLOGY, BLACKWELL PUBLISHING, OXFORD, GB, vol. 94, 1 January 1998 (1998-01-01), pages 88-93, XP002175545, ISSN: 0019-2805, DOI: 10.1046/J.1365-2567.1998.00481.X
- LENTZ M R: "THE ROLE OF THERAPEUTIC APHERESIS IN THE TREATMENT OF CANCER: A REVIEW", THERAPEUTIC APHERESIS, BLACKWELL SCIENCE, MALDEN, MA, US, vol. 3, no. 1, 1 February 1999 (1999-02-01), pages 40-49, XP001015967, ISSN: 1091-6660, DOI: 10.1046/J.1526-0968.1999.00147.X
- SELINSKY C L ET AL: "SOLUBLE TUMOR NECROSIS FACTOR RECEPTOR TYPE I ENHANCES TUMOR DEVELOPMENT AND PERSISTENCE IN VIVO", CELLULAR IMMUNOLOGY, ACADEMIC PRESS, SAN DIEGO, CA, US, vol. 200, no. 2, 15 March 2000 (2000-03-15), pages 81-87, XP001015929, ISSN: 0008-8749, DOI: 10.1006/CIMM.2000.1622
- BARBARA JEFFREY A J ET AL: "Tumour necrosis factor-alpha (TNF-alpha): The good, the bad and potentially very effective", IMMUNOLOGY AND CELL BIOLOGY, vol. 74, no. 5, 1996, pages 434-443, XP009155763, ISSN: 0818-9641
- BARBARA J.A.: 'Dissociation of TNF-alpha cytotoxic and proinflammatory activities by p55 receptor- and p75 receptor-selective TNF-alpha mutants' EMBO J. vol. 13, no. 4, 15 February 1994, pages 843 - 850, XP008126717
- ZHANG X M ET AL: "SITE-DIRECTED MUTATIONAL ANALYSIS OF HUMAN TUMOR NECROSIS FACTOR-ALPHA RECEPTOR BINDING SITE AND STRUCTURE-FUNCTIONAL RELATIONSHIP", JOURNAL OF BIOLOGICAL CHEMISTRY, AMERICAN SOCIETY FOR BIOCHEMISTRY AND MOLECULAR BIOLOGY, US, vol. 267, no. 33, 25 November 1992 (1992-11-25), pages 24069-24075, ISSN: 0021-9258

### SEQUENCE LISTING

<110> CYTOLOGIC, INC. MARK DOUGLAS HOWELL
<120> METHOD FOR ENHANCING IMMUNE RESPONSES IN MAMMALS
<130> 03325-0002P1
<140> 11/234,057
   <141> 2005-09-22
<160> 43
<170> FastSEQ for Windows Version 4.0
<210> 1
   <211> 157
   <212> PRT
   <213> Artificial Sequence
<220>
   <223> Chemically synthesized TNFalpha conserved amino acids across species
<220>
   <221> VARIANT
   <222> 1-157
   <223> Xaa=any amino acid
<400> 1
   Xaa Arg Ser Ser Ser Xaa Xaa Xaa Ser Xaa Lys Pro Val Ala His Val
    1               5                  10                  15
   Val Ala Asn Xaa Xaa Xaa Xaa Xaa Gln Leu Xaa Trp Xaa Xaa Xaa Xaa
               20                  25                  30
   Ala Asn Ala Leu Xaa Ala Asn Gly Xaa Xaa Leu Xaa Asp Asn Gln Leu
           35                  40                  45
   Xaa Val Pro Xaa Xaa Gly Leu Tyr Leu Ile Tyr Ser Gln Val Leu Phe
       50                  55                  60
   Xaa Gly Xaa Gly Cys Pro Xaa Xaa Xaa Xaa Xaa Leu Thr His Thr Xaa
   65                  70                  75                  80
   Ser Arg Xaa Ala Xaa Ser Tyr Xaa Xaa Lys Val Asn Xaa Leu Ser Ala
                   85                  90                  95
   Ile Lys Ser Pro Cys Xaa Xaa Xaa Thr Pro Glu Xaa Ala Glu Xaa Lys
               100                 105                 110
   Pro Trp Tyr Glu Pro Ile Tyr Xaa Gly Gly Val Phe Gln Leu Glu Lys
           115                 120                 125
   Xaa Asp Xaa Leu Ser Xaa Glu Xaa Asn Xaa Pro Xaa Tyr Leu Asp Xaa
       130                 135                 140
   Ala Glu Ser Gly Gln Val Tyr Phe Gly Xaa Ile Ala Leu
   145                 150                 155
<210> 2
   <211> 157
   <212> PRT
   <213> Homo sapiens
<400> 2
<210> 3
   <211> 157
   <212> PRT
   <213> Artificial Sequence
<220>
   <223> Chemically synthesized TNFalpha mutein
<400> 3
   Val Arg Ser Ser Ser Arg Thr Pro Ser Asp Lys Pro Val Ala His Val
    1              5                   10                  15
   Val Ala Asn Pro Gln Ala Glu Gly Gln Leu Gln Trp Leu Asn Pro Arg
               20                  25                  30
   Ala Asn Ala Leu Leu Ala Asn Gly Val Glu Leu Arg Asp Asn Gln Leu
           35                  40                  45
   Val Val Pro Ser Glu Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Ile Ala Val Ser Tyr Gln Thr Lys Val Asn Leu Leu Ser Ala
                   85                  90                  95
   Ile Lys Ser Pro Cys Gln Arg Glu Thr Pro Glu Gly Ala Glu Ala Lys
               100                 105                 110
   Pro Trp Tyr Glu Pro Ile Tyr Leu Gly Gly Val Phe Gln Leu Glu Lys
           115                 120                 125
   Gly Asp Arg Leu Ser Ala Glu Ile Asn Arg Pro Asp Tyr Leu Asp Phe
       130                 135                 140
   Ala Glu Ser Gly Gln Val Tyr Phe Gly Ile Ile Ala Leu
   145                 150                 155
<210> 4
   <211> 157
   <212> PRT
   <213> Artificial Sequence
<220>
   <223> Chemically synthesized TNFalpha mutein
<400> 4
   Val Arg Ser Ser Ser Arg Thr Pro Ser Asp Lys Pro Val Ala His Val
    1               5                  10                  15
   Val Ala Asn Pro Gln Ala Glu Gly Gln Leu Gln Trp Leu Asn Arg Arg
               20                  25                  30
   Ala Tyr Ala Leu Leu Ala Asn Gly Val Glu Leu Arg Asp Asn Gln Leu
           35                  40                  45
   Val Val Pro Ser Glu Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Ile Ala Val Ser Tyr Gln Thr Lys Val Asn Leu Leu Ser Ala
                   85                  90                  95
   Ile Lys Ser Pro Cys Gln Arg Glu Thr Pro Glu Gly Ala Glu Ala Lys
               100                 105                 110
   Pro Trp Tyr Glu Pro Ile Tyr Leu Gly Gly Val Phe Gln Leu Glu Lys
           115                 120                 125
   Gly Asp Arg Leu Ser Ala Glu Ile Asn Arg Pro Asp Tyr Leu Asp Phe
       130                 135                 140
   Ala Glu Ser Gly Gln Val Tyr Phe Gly Ile Ile Ala Leu
   145                 150                 155
<210> 5
   <211> 157
   <212> PRT
   <213> Artificial Sequence
<220>
   <223> Chemically synthesized TNFalpha mutein
<400> 5
   Val Arg Ser Ser Ser Arg Thr Pro Ser Asp Lys Pro Val Ala His Val
    1               5                  10                  15
   Val Ala Asn Pro Gln Ala Glu Gly Gln Leu Gln Trp Leu Asn Arg Arg
               20                  25                  30
   Ala Asn Ala Leu Leu Ala Asn Gly Val Glu Leu Arg Asp Asn Gln Leu
           35                  40                  45
   Val Val Pro Ser Glu Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Ile Ala Val Ser Tyr Gln Thr Lys Val Asn Leu Leu Ser Ala
                   85                  90                  95
   Ile Lys Ser Pro Cys Gln Arg Glu Thr Pro Glu Gly Ala Glu Ala Lys
               100                 105                 110
   Pro Trp Tyr Glu Leu Ile Tyr Leu Gly Gly Val Phe Gln Leu Glu Lys
           115                 120                 125
   Gly Asp Arg Leu Ser Ala Glu Ile Asn Arg Pro Asp Tyr Leu Asp Phe
       130                 135                 140
   Ala Glu Ser Gly Gln Val Tyr Phe Gly Ile Ile Ala Leu
   145                 150                 155
<210> 6
   <211> 157
   <212> PRT
   <213> Artificial Sequence
<220>
   <223> Chemically synthesized TNFalpha mutein
<400> 6
   Val Arg Ser Ser Ser Arg Thr Pro Ser Asp Lys Pro Val Ala His Val
    1               5                  10                  15
   Val Ala Asn Pro Gln Ala Glu Gly Gln Leu Gln Trp Leu Asn Arg Arg
               20                  25                  30
   Ala Asn Ala Leu Leu Ala Asn Gly Val Glu Leu Arg Asp Asn Gln Leu
           35                  40                  45
   Val Val Pro Ser Glu Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Ile Ala Val Ser Tyr Gln Thr Lys Val Asn Leu Leu Ser Ala
                   85                  90                  95
   Ile Lys Ser Pro Cys Gln Arg Glu Thr Pro Glu Gly Ala Glu Ala Lys
               100                 105                 110
   Pro Trp Tyr Glu Pro Ile Tyr Leu Gly Gly Val Phe Gln Leu Glu Lys
           115                 120                 125
   Gly Asp Arg Leu Ser Ala Glu Ile Asn Arg Pro Asp Tyr Leu Asp Phe
       130                 135                 140
   Ala Glu Tyr Gly Gln Val Tyr Phe Gly Ile Ile Ala Leu
   145                 150                 155
<210> 7
   <211> 157
   <212> PRT
   <213> Artificial Sequence
<220>
   <223> Chemically synthesized TNFalpha mutein
<400> 7
   Val Arg Ser Ser Ser Arg Thr Pro Ser Asp Lys Pro Val Ala His Val
    1               5                  10                  15
   Val Ala Asn Pro Gln Ala Glu Gly Gln Leu Gln Trp Leu Asn Arg Arg
               20                  25                  30
   Ala Asn Ala Leu Leu Ala Asn Gly Val Glu Leu Arg Asp Asn Gln Leu
           35                  40                  45
   Val Val Pro Ser Glu Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Ile Ala Val Ser Tyr Gln Thr Lys Val Asn Leu Leu Tyr Ala
                   85                  90                  95
   Ile Lys Ser Pro Cys Gln Arg Glu Thr Pro Glu Gly Ala Glu Ala Lys
               100                 105                 110
   Pro Trp Tyr Glu Pro Ile Tyr Leu Gly Gly Val Phe Gln Leu Glu Lys
           115                 120                 125
   Gly Asp Arg Leu Ser Ala Glu Ile Asn Arg Pro Asp Tyr Leu Asp Phe
       130                 135                 140
   Ala Glu Ser Gly Gln Val Tyr Phe Gly Ile Ile Ala Leu
   145                 150                 155
<210> 8
   <211> 157
   <212> PRT
   <213> Artificial Sequence
<220>
   <223> Chemically synthesized TNFalpha mutein
<400> 8
   Val Arg Ser Ser Ser Arg Thr Pro Ser Asp Lys Pro Val Ala His Val
    1               5                  10                  15
   Val Ala Asn Pro Gln Ala Glu Gly Gln Leu Gln Trp Leu Asn Arg Arg
               20                  25                  30
   Ala Asn Ala Leu Leu Ala Asn Gly Val Glu Leu Arg Asp Asn Gln Leu
           35                  40                  45
   Val Val Pro Ser Glu Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Ile Ala Val Ser Tyr Gln Thr Lys Val Asn Leu Leu Ser Ala
                   85                  90                  95
   Ile Lys Ser Pro Cys Gln Arg Glu Thr Pro Glu Gly Ala Glu Ala Lys
               100                 105                 110
   Pro Trp Phe Glu Pro Ile Tyr Leu Gly Gly Val Phe Gln Leu Glu Lys
           115                 120                 125
   Gly Asp Arg Leu Ser Ala Glu Ile Asn Arg Pro Asp Tyr Leu Asp Phe
       130                 135                 140
   Ala Glu Ser Gly Gln Val Tyr Phe Gly Ile Ile Ala Leu
   145                 150                 155
<210> 9
   <211> 157
   <212> PRT
   <213> Artificial Sequence
<220> >
   <223> Representative consensus TNFalpha mutein sequence that would be chemically synthesized
<220>
   <221> VARIANT
   <222> 1
   <223> Xaa=Leu or Val
<220>
   <221> VARIANT
   <222> 6
   <223> Xaa=Gln or Arg
<220>
   <221> VARIANT
   <222> 7
   <223> Xaa=Asn, Ala or Thr
<220>
   <221> VARIANT
   <222> 8
   <223> Xaa=Ser, Leu or Pro and may or may not be present
<220>
   <221> VARIANT
   <222> 10
   <223> Xaa=Asp or Asn
<220>
   <221> VARIANT
   <222> 20
   <223> Xaa=His, Pro, Leu Ile or Val
<220>
   <221> VARIANT
   <222> 21
   <223> Xaa=Gln,Glu,Ser, Asn or Lys
<220>
   <221> VARIANT
   <222> 22
   <223> Xaa=Val, Ala or Ser
<220>
   <221> VARIANT
   <222> 23
   <223> Xaa=Glu or Pro
<220>
   <221> VARIANT
   <222> 24
   <223> Xaa=Glu or Gly
<220>
   <221> VARIANT
   <222> 27
   <223> Xaa=Glu, Gln or Arg
<220>
   <221> VARIANT
   <222> 29
   <223> Xaa=Leu, Gly,Trp or Gln
<220>
   <221> VARIANT
   <222> 30
   <223> Xaa=Ser, Asp, or Asn
<220>
   <221> VARIANT
   <222> 31
   <223> Xaa=Gln, Arg, Ser or Gly
<220>
   <221> VARIANT
   <222> 32
   <223> Xaa=Arg or Tyr
<220>
   <221> VARIANT
   <222> 37
   <223> Xaa=Leu, Met or Lys
<220>
   <221> VARIANT
   <222> 41
   <223> Xaa=Met or Val
<220>
   <221> VARIANT
   <222> 42-154
   <223> Xaa=any amino acid
<400> 9
   Xaa Arg Ser Ser Ser Xaa Xaa Xaa Ser Xaa Lys Pro Val Ala His Val
    1               5                  10                  15
   Val Ala Asn Xaa Xaa Xaa Xaa Xaa Gln Leu Xaa Trp Xaa Xaa Xaa Xaa
                   20                  25                  30
   Ala Asn Ala Leu Xaa Ala Asn Gly Xaa Xaa Leu Xaa Asp Asn Gln Leu
           35                  40                  45
   Xaa Val Pro Xaa Xaa Gly Leu Tyr Leu Ile Tyr Ser Gln Val Leu Phe
       50                  55                  60
   Xaa Gly Xaa Gly Cys Pro Xaa Xaa Xaa Xaa Xaa Leu Thr His Thr Xaa
   65                  70                  75                  80
   Ser Arg Xaa Ala Xaa Ser Tyr Xaa Xaa Lys Val Asn Xaa Leu Ser Ala
                   85                  90                  95
   Ile Lys Ser Pro Cys Xaa Xaa Xaa Thr Pro Glu Xaa Ala Glu Xaa Lys
               100                 105                 110
   Pro Trp Tyr Glu Pro Ile Tyr Xaa Gly Gly Val Phe Gln Leu Glu Lys
           115                 120                 125
   Xaa Asp Xaa Leu Ser Xaa Glu Xaa Asn Xaa Pro Xaa Tyr Leu Asp Xaa
       130                 135                 140
   Ala Glu Ser Gly Gln Val Tyr Phe Gly Xaa Ile Ala Leu
   145                 150                 155
<210> 10
   <211> 156
   <212> PRT
   <213> Mus musculus
<400> 10
   Leu Arg Ser Ser Ser Gln Asn Ser Ser Asp Lys Pro Val Ala His Val
    1               5                  10                  15
   Val Ala Asn His Gln Val Glu Glu Gln Leu Glu Trp Leu Ser Gln Arg
               20                  25                  30
   Ala Asn Ala Leu Leu Ala Asn Gly Met Asp Leu Lys Asp Asn Gln Leu
           35                  40                  45
   Val Val Pro Ala Asp Gly Leu Tyr Leu Val Tyr Ser Gln Val Leu Phe
       50                  55                  60
   Lys Gly Gln Gly Cys Pro Asp Val Val Leu Leu Thr His Thr Val Ser
   65                  70                  75                  80
   Arg Phe Ala Ile Ser Tyr Gln Glu Lys Val Asn Leu Leu Ser Ala Val
                   85                  90                  95
   Lys Ser Pro Cys Pro Lys Asp Thr Pro Glu Gly Ala Glu Leu Lys Pro
               100                 105                 110
   Trp Tyr Glu Pro Ile Tyr Leu Gly Gly Val Phe Gln Leu Glu Lys Gly
           115                 120                 125
   Asp Gln Leu Ser Ala Glu Val Asn Leu Pro Lys Tyr Leu Asp Phe Ala
       130                 135                 140
   Glu Ser Gly Gln Val Tyr Phe Gly Val Ile Ala Leu
   145                 150                 155
<210> 11
   <211> 156
   <212> PRT
   <213> Rattus sp.
<400> 11
   Leu Arg Ser Ser Ser Gln Asn Ser Ser Asp Lys Pro Val Ala His Val
    1               5                  10                  15
   Val Ala Asn His Gln Ala Glu Glu Gln Leu Glu Trp Leu Ser Gln Arg
               20                  25                  30
   Ala Asn Ala Leu Leu Ala Asn Gly Met Asp Leu Lys Asp Asn Gln Leu
           35                  40                  45
   Val Val Pro Ala Asp Gly Leu Tyr Leu Ile Tyr Ser Gln Val Leu Phe
       50                  55                  60
   Lys Gly Gln Gly Cys Pro Asp Tyr Val Leu Leu Thr His Thr Val Ser
   65                  70                  75                  80
   Arg Phe Ala Ile Ser Tyr Gln Glu Lys Val Ser Leu Leu Ser Ala Ile
                   85                  90                  95
   Lys Ser Pro Cys Pro Lys Asp Thr Pro Glu Gly Ala Glu Leu Lys Pro
               100                 105                 110
   Trp Tyr Glu Pro Met Tyr Leu Gly Gly Val Phe Gln Leu Glu Lys Gly
           115                 120                 125
   Asp Leu Leu Ser Ala Glu Val Asn Leu Pro Lys Tyr Leu Asp Ile Thr
       130                 135                 140
   Glu Ser Gly Gln Val Tyr Phe Gly Val Ile Ala Leu
   145                 150                 155
<210> 12
   <211> 156
   <212> PRT
   <213> Oryctolagus cuniculus
<400> 12
   Leu Arg Ser Ala Ser Arg Ala Leu Ser Asp Lys Pro Leu Ala His Val
    1               5                  10                  15
   Val Ala Asn Pro Gln Val Glu Gly Gln Leu Gln Trp Leu Ser Gln Arg
               20                  25                  30
   Ala Asn Ala Leu Leu Ala Asn Gly Met Lys Leu Thr Asp Asn Gln Leu
           35                  40                  45
   Val Val Pro Ala Asp Gly Leu Tyr Leu Ile Tyr Ser Gln Val Leu Phe
       50                  55                  60
   Ser Gly Gln Gly Cys Arg Ser Tyr Val Leu Leu Thr His Thr Val Ser
   65                  70                  75                  80
   Arg Phe Ala Val Ser Tyr Pro Asn Lys Val Asn Leu Leu Ser Ala Ile
                   85                  90                  95
   Lys Ser Pro Cys His Arg Glu Thr Pro Glu Glu Ala Glu Pro Met Ala
               100                 105                 110
   Trp Tyr Glu Pro Ile Tyr Leu Gly Gly Val Phe Gln Leu Glu Lys Gly
           115                 120                 125
   Asp Arg Leu Ser Thr Glu Val Asn Gln Pro Glu Tyr Leu Asp Leu Ala
       130                 135                 140
   Glu Ser Gly Gln Val Tyr Phe Gly Ile Ile Ala Leu
   145                 150                 155
<210> 13
   <211> 157
   <212> PRT
   <213> Felis catus
<400> 13
   Leu Arg Ser Ser Ser Arg Thr Pro Ser Asp Lys Pro Val Ala His Val
   1               5                   10                  15
   Val Ala Asn Pro Glu Ala Glu Gly Gln Leu Gln Arg Leu Ser Arg Arg
               20                  25                  30
   Ala Asn Ala Leu Leu Ala Asn Gly Val Glu Leu Thr Asp Asn Gln Leu
           35                  40                  45
   Lys Val Pro Ser Asp Gly Leu Tyr Leu Ile Tyr Ser Gln Val Leu Phe
       50                  55                  60
   Thr Gly Gln Gly Cys Pro Ser Thr His Val Leu Leu Thr His Ala Ile
   65                  70                  75                  80
   Ser Arg Phe Ala Val Ser Tyr Gln Thr Lys Val Asn Leu Leu Ser Ala
                   85                  90                  95
   Ile Lys Ser Pro Cys Gln Arg Glu Thr Pro Glu Gly Ala Glu Ala Lys
               100                 105                 110
   Pro Trp Tyr Glu Pro Ile Tyr Leu Gly Gly Val Phe Gln Leu Glu Lys
           115                 120                 125
   Gly Asp Arg Leu Ser Thr Glu Ile Asn Leu Pro Ala Tyr Leu Asp Phe
       130                 135                 140
   Ala Glu Ser Gly Gln Val Tyr Phe Gly Ile Ile Ala Leu
   145                 150                 155
<210> 14
   <211> 157
   <212> PRT
   <213> Canis familiaris
<400> 14
   Val Leu Ser Ser Ser Arg Thr Pro Ser Asp Lys Pro Val Ala His Val
   1               5                   10                  15
   Val Ala Asn Pro Glu Ala Glu Gly Gln Leu Gln Trp Leu Ser Arg Arg
               20                  25                  30
   Ala Asn Ala Leu Leu Ala Asn Gly Val Glu Leu Thr Asp Asn Gln Leu
           35                  40                  45
   Ile Val Pro Ser Asp Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Phe Ala Val Ser Tyr Gln Thr Lys Val Asn Leu Leu Ser Ala
                   85                  90                  95
   Ile Lys Ser Pro Cys Gln Arg Glu Thr Pro Glu Gly Thr Glu Ala Lys
               100                 105                 110
   Pro Trp Tyr Glu Pro Ile Tyr Leu Gly Gly Val Phe Gln Leu Glu Lys
           115                 120                 125
   Gly Asp Arg Leu Ser Ala Glu Ile Asn Leu Pro Asn Tyr Leu Asp Phe
       130                 135                 140
   Ala Glu Ser Gly Gln Val Tyr Phe Gly Ile Ile Ala Leu
   145                 150                 155
<210> 15
   <211> 157
   <212> PRT
   <213> Ovis aries
<400> 15
   Leu Arg Ser Ser Ser Gln Ala Ser Asn Asn Lys Pro Val Ala His Val
    1               5                  10                  15
   Val Ala Asn Leu Ser Ala Pro Gly Gln Leu Arg Trp Gly Asp Ser Tyr
               20                  25                  30
   Ala Asn Ala Leu Met Ala Asn Gly Val Glu Leu Lys Asp Asn Gln Leu
           35                  40                  45
   Val Val Pro Thr Asp Gly Leu Tyr Leu Ile Tyr Ser Gln Val Leu Phe
       50                  55                  60
   Arg Gly His Gly Cys Pro Ser Thr Pro Leu Phe Leu Thr His Thr Ile
   65                  70                  75                  80
   Ser Arg Ile Ala Val Ser Tyr Gln Thr Lys Val Asn Ile Leu Ser Ala
                   85                  90                  95
   Ile Lys Ser Pro Cys His Arg Glu Thr Leu Glu Gly Ala Glu Ala Lys
               100                 105                 110
   Pro Trp Tyr Glu Pro Ile Tyr Gln Gly Gly Val Phe Gln Leu Glu Lys
           115                 120                 125
   Gly Asp Arg Leu Ser Ala Glu Ile Asn Leu Pro Glu Tyr Leu Asp Tyr
       130                 135                 140
   Ala Glu Ser Gly Gln Val Tyr Phe Gly Ile Ile Ala Leu
   145                 150                 155
<210> 16
   <211> 156
   <212> PRT
   <213> Capra hircus
<400> 16
   Leu Arg Ser Ser Ser Gln Ala Ser Ser Asn Lys Pro Val Ala His Val
    1               5                  10                  15
   Val Ala Asn Ile Ser Ala Pro Gly Gln Leu Arg Trp Gly Asp Ser Tyr
               20                  25                  30
   Ala Asn Ala Leu Lys Ala Asn Gly Val Glu Leu Lys Asp Asn Gln Leu
           35                  40                  45
   Val Val Pro Thr Asp Gly Leu Tyr Leu Ile Tyr Ser Gln Val Leu Phe
       50                  55                  60
   Arg Gly His Gly Cys Pro Ser Thr Pro Leu Phe Leu Thr His Thr Ile
   65                  70                  75                  80
   Ser Arg Ile Ala Val Ser Tyr Gln Thr Lys Val Asn Ile Leu Ser Ala
                   85                  90                  95
   Ile Lys Ser Pro Cys His Arg Glu Thr Pro Glu Ala Glu Ala Lys Pro
               100                 105                 110
   Trp Tyr Glu Pro Ile Tyr Gln Gly Gly Val Glu Gln Leu Glu Lys Gly
           115                 120                 125
   Asp Arg Leu Ser Ala Glu Ile Asn Gln Pro Glu Tyr Leu Asp Tyr Ala
       130                 135                 140
   Glu Ser Gly Gln Val Tyr Phe Gly Ile Ile Ala Leu
   145                 150                 155
<210> 17
   <211> 157
   <212> PRT
   <213> Equus caballus
<400> 17
   Leu Arg Ser Ser Ser Arg Thr Pro Ser Asp Lys Pro Val Ala His Val
    1               5                  10                  15
   Val Ala Asn Pro Gln Ala Glu Gly Gln Leu Gln Trp Leu Ser Gly Arg
               20                  25                  30
   Ala Asn Ala Leu Leu Ala Asn Gly Val Lys Leu Thr Asp Asn Gln Leu
           35                  40                  45
   Val Val Pro Leu Asp Gly Leu Tyr Leu Ile Tyr Ser Gln Val Leu Phe
       50                  55                  60
   Lys Gly Gln Gly Cys Pro Ser Thr His Val Leu Leu Thr His Thr Ile
   65                  70                  75                  80
   Ser Arg Leu Ala Val Ser Tyr Pro Ser Lys Val Asn Leu Leu Ser Ala
                   85                  90                  95
   Ile Lys Ser Pro Cys His Thr Glu Ser Pro Glu Gln Ala Glu Ala Lys
               100                 105                 110
   Pro Trp Tyr Glu Pro Ile Tyr Leu Gly Gly Val Phe Gln Leu Glu Lys
           115                 120                 125
   Gly Asp Gln Leu Ser Ala Glu Ile Asn Gln Pro Asn Tyr Leu Asp Phe
       130                 135                 140
   Ala Glu Ser Gly Gln Val Tyr Phe Gly Ile Ile Ala Leu
   145                 150                 155
<210> 18
   <211> 156
   <212> PRT
   <213> Box taurus
<400> 18
   Leu Arg Ser Ser Ser Gln Ala Ser Ser Asn Lys Pro Val Ala His Val
    1               5                  10                  15
   Val Ala Asp Ile Asn Ser Pro Gly Gln Leu Arg Trp Trp Asp Ser Tyr
               20                  25                  30
   Ala Asn Ala Leu Met Ala Asn Gly Val Gln Leu Glu Asp Asn Gln Leu
           35                  40                  45
   Val Val Pro Ala Glu Gly Leu Tyr Leu Ile Tyr Ser Gln Val Leu Phe
       50                  55                  60
   Arg Gly Gln Gly Cys Pro Pro Pro Pro Val Leu Thr His Thr Ile Ser
   65                  70                  75                  80
   Arg Ile Ala Val Ser Tyr Gln Thr Lys Val Asn Ile Leu Ser Ala Ile
                   85                  90                  95
   Lys Ser Pro Cys His Arg Glu Thr Pro Glu Trp Ala Glu Ala Lys Pro
               100                 105                 110
   Trp Tyr Glu Pro Ile Tyr Gln Gly Gly Val Phe Gln Leu Glu Lys Asp
           115                 120                 125
   Asp Arg Leu Ser Ala Glu Ile Asn Leu Pro Asp Tyr Leu Asp Tyr Ala
       130                 135                 140
   Glu Ser Gly Gln Val Tyr Phe Gly Ile Ile Ala Leu
   145                 150                 155
<210> 19
   <211> 156
   <212> PRT
   <213> Sus scrofa
<400> 19
   Leu Arg Ser Ser Ser Gln Thr Ser Asp Lys Pro Val Ala His Val Val
   1 5 10 15
   Ala Asn Val Lys Ala Glu Gly Gln Leu Gln Trp Gln Ser Gly Tyr Ala
   20 25 30
   Asn Ala Leu Leu Ala Asn Gly Val Lys Leu Lys Asp Asn Gln Leu Val
   35 40 45
   Val Pro Thr Asp Gly Leu Tyr Leu Ile Tyr Ser Gln Val Leu Phe Arg
   50 55 60
   Gly Gln Gly Cys Pro Ser Thr Asn Val Phe Leu Thr His Thr Ile Ser
   65 70 75 80
   Arg Ile Ala Val Ser Tyr Gln Thr Lys Val Asn Leu Leu Ser Ala Ile
   85 90 95
   Lys Ser Pro Cys Gln Arg Glu Thr Pro Glu Gly Ala Glu Ala Lys Pro
   100 105 110
   Trp Tyr Glu Pro Ile Tyr Leu Gly Gly Val Phe Gln Leu Glu Lys Asp
   115 120 125
   Asp Arg Leu Ser Ala Glu Ile Asn Leu Pro Asp Tyr Leu Asp Phe Ala
   130 135 140
   Glu Ser Gly Gln Val Tyr Phe Gly Ile Ile Ala Leu
   145 150 155
<210> 20
   <211> 6
   <212> PRT
   <213> Homo sapiens
<400> 20
   Asp Phe Ala Glu Ser Gly
   1 5
<210> 21
   <211> 5
   <212> PRT
   <213> Artificial Sequence
<220>
   <223> Chemically synthesized variant of TNFalpha mutein consensus sequence
<400> 21
   His Gln Val Glu Glu
   1 5
<210> 22
   <211> 5
   <212> PRT
   <213> Artificial Sequence
<220>
   <223> Chemically synthesized variant of TNFalpha mutein consensus sequence
<400> 22
   His Gln Ala Glu Glu
   1 5
<210> 23
   <211> 5
   <212> PRT
   <213> Artificial Sequence
<220>
   <223> Chemically synthesized variant of TNFalpha mutein consensus sequence
<400> 23
   Pro Gln Val Glu Gly
   1 5
<210> 24
   <211> 5
   <212> PRT
   <213> Artificial Sequence
<220>
   <223> Chemically synthesized variant of TNFalpha mutein consensus sequence
<400> 24
   Pro Glu Ala Glu Gly
   1 5
<210> 25
   <211> 5
   <212> PRT
   <213> Artificial Sequence
<220>
   <223> Chemically synthesized variant of TNFalpha mutein consensus sequence
<400> 25
   Leu Ser Ala Pro Gly
   1 5
<210> 26
   <211> 5
   <212> PRT
   <213> Artificial Sequence
<220>
   <223> Chemically synthesized variant of TNFalpha mutein consensus sequence
<400> 26
   Ile Ser Ala Pro Gly
   1 5
<210> 27
   <211> 5
   <212> PRT
   <213> Artificial Sequence
<220>
   <223> Chemically synthesized variant of TNFalpha mutein consensus sequence
<400> 27
   Pro Gln Ala Glu Gly
   1 5
<210> 28
   <211> 5
   <212> PRT
   <213> Artificial Sequence
<220>
   <223> Chemically synthesized variant of TNFalpha mutein consensus sequence
<400> 28
   Ile Asn Ser Pro Gly
   1 5
<210> 29
   <211> 5
   <212> PRT
   <213> Artificial Sequence
<220>
   <223> Chemically synthesized variant of TNFalpha mutein consensus sequence
<400> 29
   Val Lys Ala Glu Gly
   1 5
<210> 30
   <211> 4
   <212> PRT
   <213> Artificial Sequence
<220>
   <223> Chemically synthesized variant of TNFalpha mutein consensus sequence
<400> 30
   Leu Ser Gln Arg
   1
<210> 31
   <211> 4
   <212> PRT
   <213> Artificial Sequence
<220>
   <223> Chemically synthesized variant of TNFalpha mutein consensus sequence
<400> 31
   Leu Ser Arg Arg
   1
<210> 32
   <211> 4
   <212> PRT
   <213> Artificial Sequence
<220>
   <223> Chemically synthesized variant of TNFalpha mutein consensus sequence
<400> 32
   Gly Asp Ser Tyr
   1
<210> 33
   <211> 4
   <212> PRT
   <213> Artificial Sequence
<220>
   <223> Chemically synthesized variant of TNFalpha mutein consensus sequence
<400> 33
   Leu Ser Gly Arg
   1
<210> 34
   <211> 4
   <212> PRT
   <213> Artificial Sequence
<220>
   <223> Chemically synthesized variant of TNFalpha mutein consensus sequence
<400> 34
   Trp Asp Ser Tyr
   1
<210> 35
   <211> 4
   <212> PRT
   <213> Artificial Sequence
<220>
   <223> Chemically synthesized variant of TNFalpha mutein consensus sequence
<400> 35
   Gln Ser Gly Tyr
   1
<210> 36
   <211> 4
   <212> PRT
   <213> Artificial Sequence
<220>
   <223> Chemically synthesized variant of TNFalpha mutein consensus sequence
<400> 36
   Leu Asn Arg Arg
   1
<210> 37
   <211> 4
   <212> PRT
   <213> Artificial Sequence
<220>
   <223> Chemically synthesized variant of TNFalpha mutein consensus sequence
<400> 37
   Asp Val Val Leu
   1
<210> 38
   <211> 4
   <212> PRT
   <213> Artificial Sequence
<220>
   <223> Chemically synthesized variant of TNFalpha mutein consensus sequence
<400> 38
   Asp Tyr Val Leu
   1
<210> 39
   <211> 4
   <212> PRT
   <213> Artificial Sequence
<220>
   <223> Chemically synthesized variant of TNFalpha mutein consensus sequence
<400> 39
   Ser Tyr Val Leu
   1
<210> 40
   <211> 4
   <212> PRT
   <213> Artificial Sequence
<220>
   <223> Chemically synthesized variant of TNFalpha mutein consensus sequence
<400> 40
   Pro Pro Pro Val
   1
<210> 41
   <211> 5
   <212> PRT
   <213> Artificial Sequence
<220>
   <223> Chemically synthesized variant of TNFalpha mutein consensus sequence
<400> 41
   Ser Thr His Val Leu
   1 5
<210> 42
   <211> 5
   <212> PRT
   <213> Artificial Sequence
<220>
   <223> Chemically synthesized variant of TNFalpha mutein consensus sequence
<400> 42
   Ser Thr Pro Leu Phe
   1 5
<210> 43
   <211> 5
   <212> PRT
   <213> Artificial Sequence
<220>
   <223> Chemically synthesized variant of TNFalpha mutein consensus sequence
<400> 43
   Ser Thr Asn Val Phe
   1 5

## Description

### BACKGROUND OF THE INVENTION

### FIELD OF THE INVENTION

This invention relates generally to the field of immunotherapy and, more specifically, to methods for enhancing host immune responses.

### BACKGROUND ART

The immune system of mammals has evolved to protect the host against the growth and proliferation of potentially deleterious agents. These agents include infectious microorganisms such as bacteria, viruses, fungi, and parasites which exist in the environment and which, upon introduction to the body of the host, can induce varied pathological conditions. Other pathological conditions may derive from agents not acquired from the environment, but rather which arise spontaneously within the body of the host. The best examples are the numerous malignancies known to occur in mammals. Ideally, the presence of these deleterious agents in a host triggers the mobilization of the immune system to effect the destruction of the agent and, thus, restore the sanctity of the host environment.

The destruction of pathogenic agents by the immune system involves a variety of effector mechanisms which can be grouped generally into two categories:
innate and specific immunity. The first line of defense is mediated by the mechanisms of innate immunity. Innate immunity does not discriminate among the myriad agents that might gain entry into the host's body. Rather, it responds in a generalized manner that employs the inflammatory response, phagocytes, and plasma-borne components such as complement and interferons. In contrast, specific immunity does discriminate among pathogenic agents. Specific immunity is mediated by B and T lymphocytes, and it serves, in large part, to amplify and focus the effector mechanisms of innate immunity.

The elaboration of an effective immune response requires contributions from both innate and specific immune mechanisms. The function of each of these arms of the immune system individually, as well as their interaction with each other, is carefully coordinated, both in a temporal/spatial manner and in terms of the particular cell types that participate. This coordination results from the actions of a number of soluble immunostimulatory mediators or "immune system stimulators" (reviewed in, Trinchieri et al., J. Cell, Biochem. 53:301-308 (1993)). Certain of these immune system stimulators initiate and perpetuate the inflammatory response and the attendant systemic sequelae. Examples of these include, but are not limited to, the proinflammatory mediators tumor necrosis factors α and *β*, interleukin-1, interleukin-6, interleukin-8, interferon-γ, and the chemokines RANTES, macrophage inflammatory proteins 1-α and 1-*β* and macrophage chemotactic and activating factor. Other immune system stimulators facilitate interactions between B and T lymphocytes of specific immunity. Examples of these include, but are not limited to, interleukin-2, interleukin-4, interleukin-5, interleukin-6, and interferon-γ. Still other immune system stimulators mediate bidirectional communication between specific immunity and innate immunity. Examples of these include, but are not limited to, interferon-7, interleukin-1, tumor necrosis factors α and *β*, and interleukin-12. All of these immune system stimulators exert their effects by binding the specific receptors on the surface of host cells, resulting in the delivery of intracellular signals that alter the function of the target cell. Cooperatively, these mediators stimulate the activation and proliferation of immune cells, recruit them to particular anatomical sites, and permit their collaboration in the elimination of the offending agent. The immune response induced in any individual is determined by the particular complement of immune system stimulators produced and by the relative abundance of each.

In contrast to the immune system stimulators described above, the immune system has evolved other soluble mediators that serve to inhibit immune responses (reviewed in Arend, Adv. Int. Med. 40:365-394 (1995)). These "immune system inhibitors" provide the immune system with the ability to dampen responses in order to prevent the establishment of a chronic inflammatory state with the potential to damage the host's tissues. Regulation of host immune function by immune system inhibitors is accomplished through a variety of mechanisms as described below.

First, certain immune system inhibitors bind directly to immune system stimulators and, thus, prevent them from binding to plasma membrane receptors on host cells. Examples of these types of immune system inhibitors include, but are not limited to, the soluble receptors for tumor necrosis factors α and *β*, interferon-γ, interleulcin-1, interleulcin-2, interleukin-4, interleukin-6, and interleukin-7.

Second, certain immune system inhibitors antagonize the binding of immune system stimulators to their receptors. By way of example, interleulcin-1 receptor antagonist is known to bind to the interleukin-1 membrane receptor. It does not deliver activation signals to the target cell but, by virtue of occupying the interleulcin-1 membrane receptor, blocks the effects of interleukin-1.

Third, particular immune system inhibitors exert their effects by binding to receptors on host cells and signaling a decrease in their production of immune system stimulators. Examples include, but are not limited to, interferon-*β*, which decreases the production of two key proinflammatory mediators, tumor necrosis factor α and interleukin-1 (Coclet-Ninin et al., Eur. Cytokine Network 8:345-349 (1997)), and interleukin-10, which suppresses the development of cell-mediated immune responses by inhibiting the production of the immune system stimulator, interleukin-12 (D'Andrea et al., J. Exp. Med. 178:1041-1048 (1993)). In addition to decreasing the production of immune system stimulators, certain immune system inhibitors also enhance the production of other immune system inhibitors. By way of example, interferon-α_{2b} inhibits interleukin-1 and tumor necrosis factor α production and increases the production of the corresponding immune system inhibitors, interleukin-1 receptor antagonist and soluble receptors for tumor necrosis factors α and *β* (Dinarello, Sem. in Oncol. 24(3 Suppl. 9):81-93 (1997).

Fourth, certain immune system inhibitors act directly on immune cells, inhibiting their proliferation and function, thereby decreasing the vigor of the immune response. By way of example, transforming growth factor-*β* inhibits a variety of immune cells and significantly limits inflammation and cell-mediated immune responses (reviewed in Letterio and Roberts, Ann. Rev. Immunol. 16:137-161 (1998)). Collectively, these various immunosuppressive mechanisms are intended to regulate the immune response, both quantitatively and qualitatively, to minimize the potential for collateral damage to the host's own tissues.

In addition to the inhibitors produced by the host's immune system for self-regulation, other immune system inhibitors are produced by infectious microorganisms. For example, many viruses produce molecules which are viral homologues of host immune system inhibitors (reviewed in Spriggs, Ann. Rev. Immunol. 14:101-130 (1996)). These include homologues of host complement inhibitors, interleukin-10, and soluble receptors for interleukin-1, tumor necrosis factors α and *β*, and interferons α, *β* and γ. Similarly, helminthic parasites produce homologues of host immune system inhibitors (reviewed in Riffkin et al., Immunol. Cell Biol. 74:564-574 (1996)), and several bacterial genera are known to produce immunosuppressive products (reviewed in, Reimann et al., Scand. J. Immunol. 31:543-546 (1990)). All of these immune system inhibitors serve to suppress the immune response during the initial stages of infection, to provide advantage to the microbe, and to enhance the virulence and chronicity of the infection.

A role for host-derived immune system inhibitors in chronic disease also has been established. In the majority of cases, this reflects a polarized T cell response during the initial infection, wherein the production of immunosuppressive mediators (i.e., interleukin-4, interleukin-10, and/or transforming growth factor-*β*) dominates over the production of immunostimulatory mediators (i.e., interleukin-2, interferon-γ, and/or tumor necrosis factor *β*) (reviewed in Lucey et al., Clin, Micro. Rev. 9:532-562 (1996)). Overproduction of immunosuppressive mediators of this type has been shown to produce chronic, non-healing pathologies in a number of medically important diseases. These include, but are not limited to, diseases resulting from infection with: 1) the parasites, *Plasnzodium falciparum* (Sarthou et al., Infect. Immun. 65:3271-3276 (1997)), *Trypanosoma cruzi* (reviewed in Laucella et al., Revista Argentina de Microbiolgia 28:99-109 (1996)), *Leishmania major* (reviewed in Etges and Muller, J. Mol. Med. 76:372-390 (1998)), and certain helminths (Riffkin et al., *supra*); 2) the intracellular bacteria, *Mycobacterium tuberculosis* (Baliko et al., FEMS Immunol. Med. Micro. 22:199-204 (1998)), *Mycobacterium avium* (Bermudez and Champsi, Infect. Immun. 61:3093-3097 (1993)), *Mycobacterium leprae* (Sieling et al., J. Immunol. 150:5501-5510 (1993)), *Mycobacterium bovis* (Kaufmann et al., Ciba Fdn. Symp. 195:123-132 (1995)), *Brucella abortus* (Fernandes and Baldwin, Infect. Immun. 63:1130-1133 (1995)), and *Wisteria monocytogenes* (Blauer et al., J. Interferon Cytokine Res. 15:105-114 (1995)), and, 3) intracellular fungus, *Candida albicans* (reviewed in Romani et al., Immunol. Res. 14:148-162 (1995)). The inability to spontaneously resolve infection is influenced by other host-derived immune system inhibitors as well. By way of example, interleukin-1 receptor antagonist and the soluble receptors for tumor necrosis factors α and *β* are produced in response to interleulcin-1 and tumor necrosis factor α and/or *β* production driven by the presence of numerous infectious agents. Examples include, but are not limited to, infections by *Plasmodium falciparum* (Jakobsen et al., Infect. Immun. 66:1654-1659 (1998); Sarthou et al., *supra*), *Mycobacteriurn tuberculosis* (Balcewicz-Sablinska et al., J. Immunol. 161:2636-2641 (1998)), and *Mycobacterium avium* (Erilcs and Emerson, Infect. Immun. 65:2100-2106 (1997)). In cases where the production of any of the aforementioned immune system inhibitors, either individually or in combination, dampens or otherwise alters immune responsiveness before the elimination of the pathogenic agent, a chronic infection may result.

In addition to this role in infectious disease, host-derived immune system inhibitors contribute also to chronic malignant disease. Compelling evidence is provided by studies of soluble tumor necrosis factor receptor Type I (sTNFRI) in cancer patients. Nanomolar concentrations of sTNFRI are synthesized by a variety of activated immune cells in cancer patients and, in many cases, by the tumors themselves (Aderka et al., Cancer Res. 51:5602-5607 (1991); Adolf and Apfler, J. Immunol. Meth. 143:127-136 (1991)). In addition, circulating sTNFRI levels often are elevated significantly in cancer patients (Aderka et al., *supra;* Kalmanti et al., Int. J. Hematol. 57:147-152 (1993); Elsasser-Beile et al., Tumor Biol. 15:17-24 (1994); Gadducci et al., Anticancer Res. 16:3125-3128 (1996); Digel et al., J. Clin. Invest. 89:1690-1693 (1992)), decline during remission and increase during advanced stages of tumor development (Aderka et al., *supra;* Kalmanti et al., *supra;* Elsasser-Beile et al., *supra;* Gadducci et al., *supra*) and, when present at high levels, correlate with poorer treatment outcomes (Aderka et al., *supra*). These observations suggest that sTNFRI aids tumor survival by inhibiting anti-tumor immune mechanisms which employ tumor necrosis factors α and/or *β* (TNF), and they argue favorably for the clinical manipulation of sTNFRI levels as a therapeutic strategy for cancer.

Direct evidence that the removal of immune system inhibitors provides clinical benefit derives from the evaluation of Ultrapheresis, a promising experimental cancer therapy (Lentz, J. Biol. Response Modif. 8:511-527 (1989); Lentz, Ther. Apheresis 3:40-49 (1999); Lentz, Jpn. J. Apheresis 16:107-114 (1997)). Ultrapheresis involves extracorporeal fractionation of plasma components by ultrafiltration. Ultrapheresis selectively removes plasma components within a defined molecular size range, and it has been shown to provide significant clinical advantage to patients presenting with a variety of tumor types. Ultrapheresis induces pronounced inflammation at tumor sites, often in less than one hour post-initiation. This rapidity suggests a role for preformed chemical and/or cellular mediators in the elaboration of this inflammatory response, and it reflects the removal of naturally occurring plasma inhibitors of that response. Indeed, immune system inhibitors of TNF α and *β*, interleukin-1, and interleukin-6 are removed by Ultrapheresis (Lentz, Ther. Apheresis 3:40-49 (1999)). Notably, the removal of sTNFRI has been correlated with the observed clinical responses (Lentz, Ther. Apheresis 3:40-49 (1999); Lentz, Jpn. J. Apheresis 16:107-114 (1997)).

Ultrapheresis is in direct contrast to more traditional approaches which have endeavored to boost immunity through the addition of immune system stimulators. Preeminent among these has been the infusion of supraphysiological levels of TNF (Sidhu and Bollon, Pharmacol. Ther. 57:79-128 (1993)); and of interleukin-2 (Maas et al., Cancer Immunol. Immunother. 36:141-148 (1993)), which indirectly stimulates the production of TNF. These therapies have enjoyed limited success (Sidhu and Bollon, *supra,* Maas et al., *supra*) due to the fact: 1) that at the levels employed they proved extremely toxic; and 2) that each increases the plasma levels of the immune system inhibitor, sTNFRI (Lantz et al., Cytokine 2:402-406 (1990); Miles et al., Brit. J. Cancer 66:1195-1199 (1992)). Together, these observations support the utility of Ultrapheresis as a biotherapeutic approach to cancer - one which involves the removal of immune system inhibitors, rather than the addition of immune system stimulators.

Although Ultrapheresis provides advantages over traditional therapeutic approaches, there are certain drawbacks that limit its clinical usefulness. Not only are immune system inhibitors removed by Ultrapheresis, but other plasma components, including beneficial ones, are removed since the discrimination between removed and retained plasma components is based solely on molecular size. An additional drawback to Ultrapheresis is the significant loss of circulatory volume during treatment, which must be offset by the infusion of replacement fluid. The most effective replacement fluid is an ultrafiltrate produced, in an identical manner, from the plasma of non-tumor bearing donors. A typical treatment regimen (15 treatments, each with the removal of approximately 7 liters of ultrafiltrate) requires over 200 liters of donor plasma for the production of replacement fluid. The chronic shortage of donor plasma, combined with the risks of infection by human immunodeficiency virus, hepatitis A, B, and C or other etiologic agents, represents a severe impediment to the widespread implementation of Ultrapheresis.

WO0143770 describes a method for enhancing immune responses in mammals by removing immune response inhibitors, i.e. sTNFRI, from biological fluids.

Because of the beneficial effects associated with the removal of immune system inhibitors, there exists a need for methods which can be used to specifically deplete those inhibitors from circulation. Such methods ideally should be specific and not remove other circulatory components, and they should not result in any significant loss of circulatory volume. The present invention satisfies these needs and provides related advantages as well.

### SUMMARY OF THE INVENTION

The present invention is defined in the claims. Provided is a method for stimulating immune responses in a mammal through the depletion of immune system inhibitors such as soluble TNF receptors present in the circulation of the mammal. The depletion of immune system inhibitors such as soluble TNF receptors can be effected by removing biological fluids from the mammal and contacting these biological fluids with a binding partner, for example, TNFα muteins, capable of selectively binding to the targeted immune system inhibitor.

Binding partners useful in these methods are TNFα muteins having specificity for soluble TNF receptors. Moreover, mixtures of TNFα muteins having specificity for one or more soluble TNF receptors can be used.

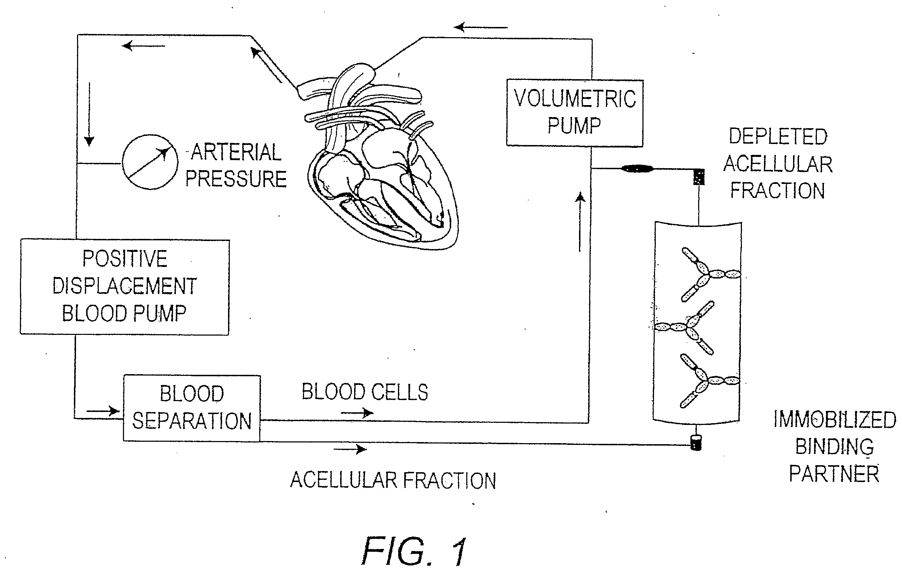

As an example, a binding partner, such as a TNFα mutein, is immobilized previously on a solid support to create an "adsorbent matrix" (Figure 1). The exposure of biological fluids to such an adsorbent matrix will permit binding by the immune system inhibitor such as soluble TNF receptor, thus, effecting a decrease in its abundance in the biological fluids. The treated biological fluid can be returned to the patient. The total volume of biological fluid to be treated and the treatment rate are parameters individualized for each patient, guided by the induction of vigorous immune responses while minimizing toxicity. The solid support (i.e., inert medium) can be composed of any material useful for such purpose, including, for example, hollow fibers, cellulose-based fibers, synthetic fibers, flat or pleated membranes, silica-based particles, macroporous beads, and the like.

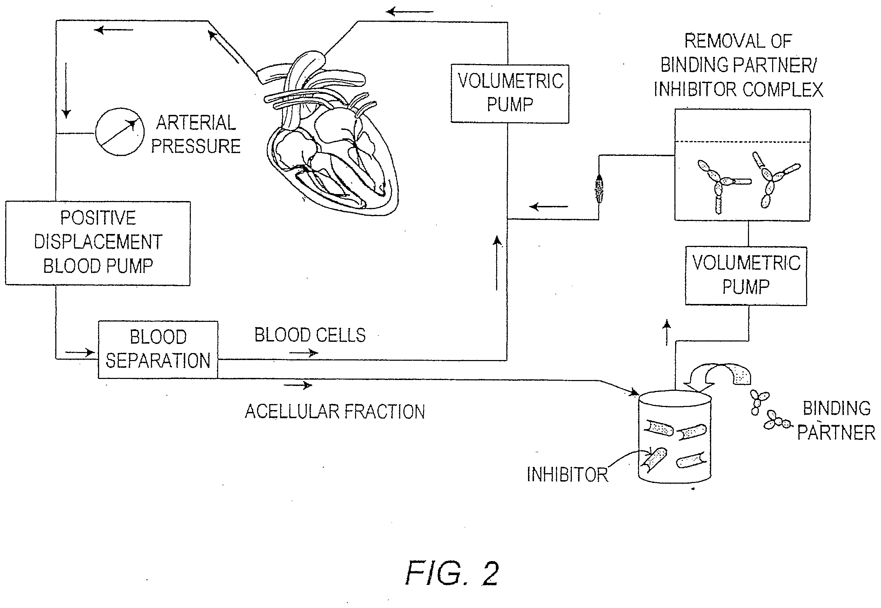

As another example, the binding partner such as TNFα mutein can be mixed with the biological fluid in a "stirred reactor" (Figure 2). The binding partner-immune system inhibitor complex then can be removed by mechanical or by chemical or biological means or methods, and the altered biological fluid can be returned to the patient.

Also provided are conjugates comprising a tumor necrosis factor α (TNFα) mutein attached to a substrate.

Further provided are apparatuses incorporating either the adsorbent matrix or the stirred reactor.

### BRIEF DESCRIPTION OF THE FIGURES

The accompanying drawings, which are incorporated in and constitute a part of this specification, illustrate several aspects of the invention and together with the description, serve to explain the principles of the invention.
Figure 1 schematically illustrates an "adsorbent matrix" configuration of an aspect of the disclosed compositions, conjugates and methods. In this example, blood is removed from the patient and separated into a cellular and an acellular component, or factions thereof. The acellular component, or fractions thereof, is exposed to the adsorbent matrix to effect the binding and, thus, depletion of a targeted immune system inhibitor such as soluble tumor necrosis factor (TNF) receptor. The altered acellular component, or fractions thereof, then is returned contemporaneously to the patient.
Figure 2 schematically illustrates a "stirred reactor" configuration of an aspect of the disclosed compositions, conjugates and methods. In this example, blood is removed from the patient and separated into a cellular and an acellular component, or fractions thereof. A binding partner such as a TNFα mutein is added to the acellular component, or fractions thereof. Subsequently, the binding partner (TNFα mutein)/immune system inhibitor (soluble TNF receptor) complex is removed by mechanical or by chemical or biological means or methods from the acellular component, or fractions thereof, and the altered biological fluid is returned contemporaneously to the patient.

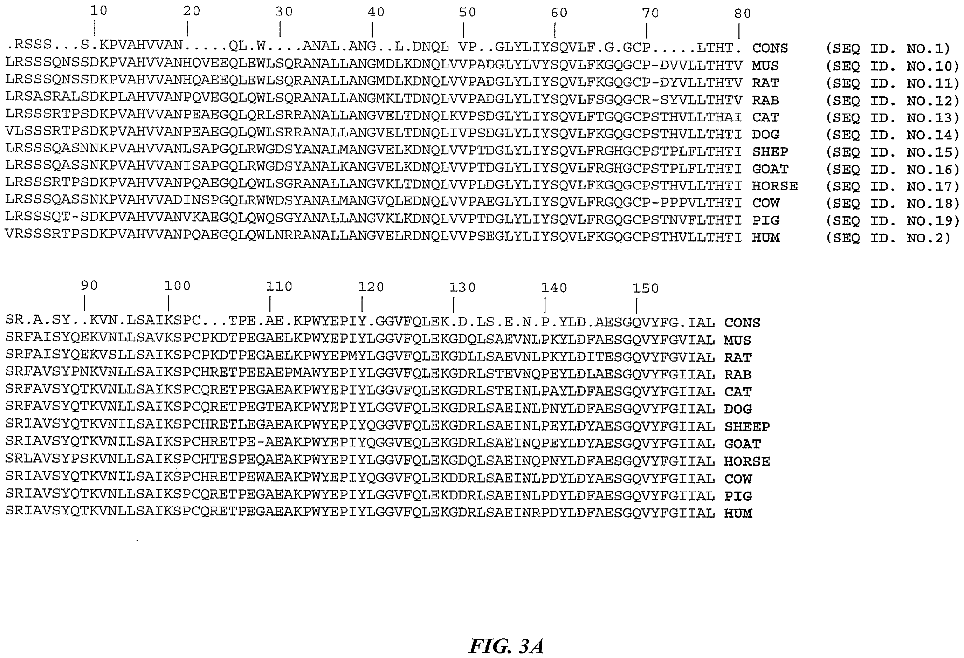

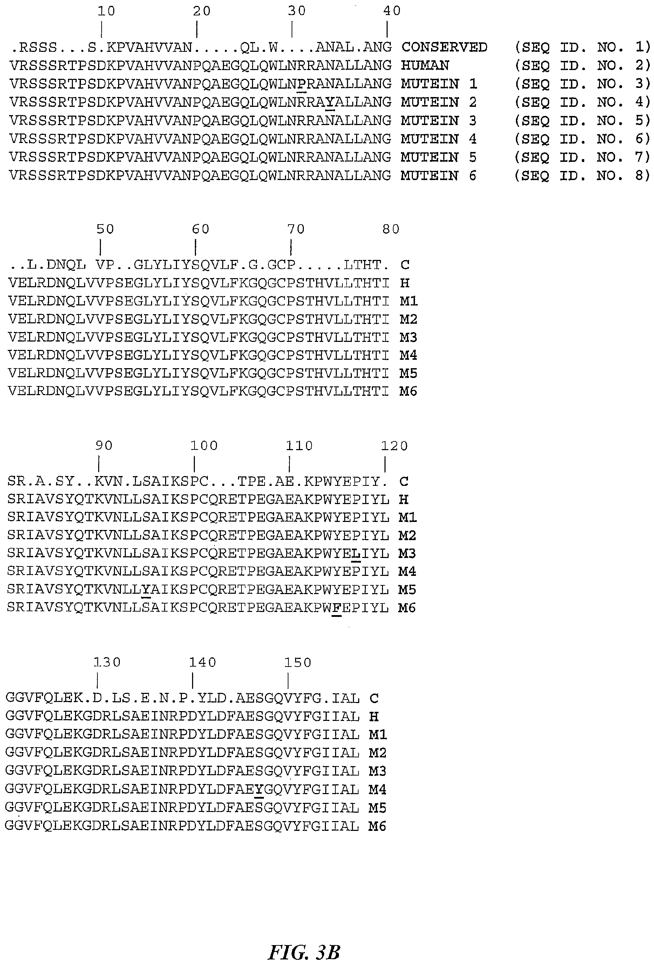

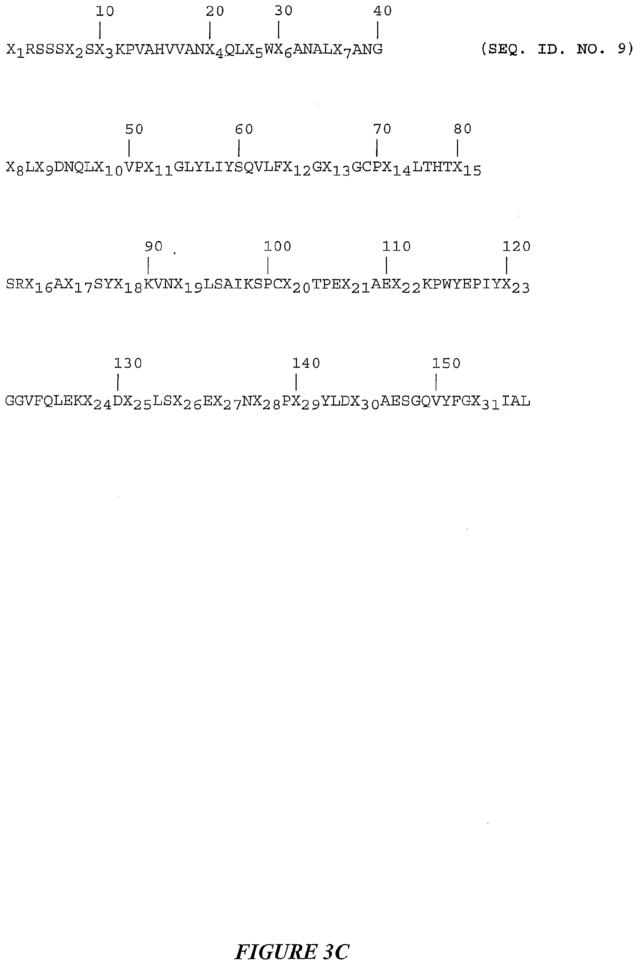

Figure 3A shows an alignment of TNFα sequences from various mammalian species (mouse, SEQ ID NO:10; rat, SEQ ID NO:11; rabbit, SEQ ID NO:12; cat, SEQ ID NO:13; dog, SEQ ID NO:14; sheep, SEQ ID NO:15; goat, SEQ ID NO:16; horse, SEQ ID NO:17; cow, SEQ ID NO:18; pig, SEQ ID NO:19; human, SEQ ID NO:2). The top sequence shows the conserved amino acids across the shown species (SEQ ID NO:1)(completely conserved or with one exception). Non-conserved amino acids are indicated by "." (taken from Van Ostade et al., Prot. Eng. 7:5-22 (1994)). Figure 3B shows an alignment of the conserved TNFα sequence with human TNFα and six representative TNFα muteins, designated mutein 1 (SEQ ID NO:3), mutein 2 (SEQ ID NO:4), mutein 3 (SEQ ID NO:5), mutein 4 (SEQ ID NO:6), mutein 5 (SEQ ID NO:7), and mutein 6 (SEQ ID NO:8). The four muteins differ from the human sequence by single amino acid substitutions, indicated with bold and underline. Figure 3C shows a representative consensus TNFα sequence (SEQ ID NO:9).

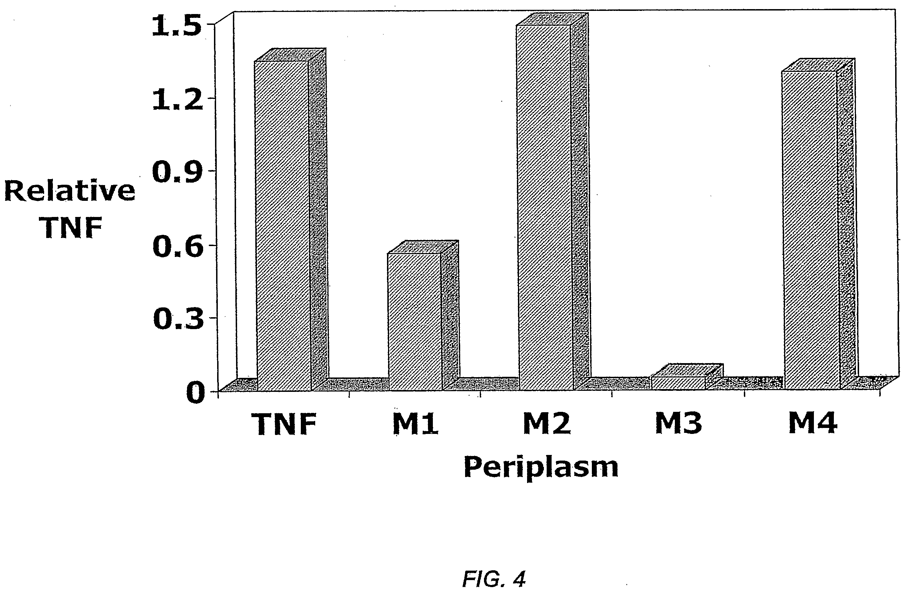

Figure 4 shows the presence of human TNFα and TNFα muteins 1, 2, 3 and 4 in periplasmic preparations of *Escherichia coli* transformed with the respective expression constructs.

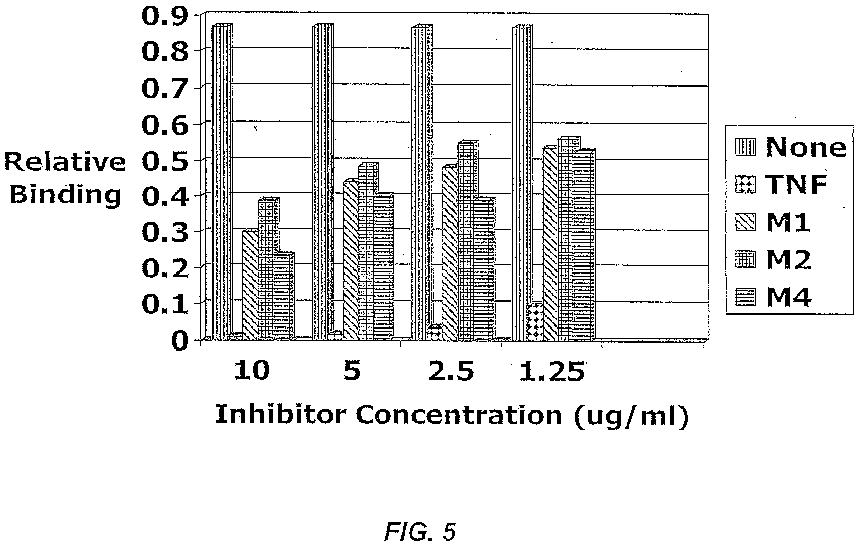

Figure 5 shows that TNFα muteins bind to sTNFRI. Wells of a microtiter plate were coated with TNFα, blocked, and incubated with STNFRI either in the presence or absence of the inhibitors, TNFα and TNFα muteins 1,2 and 4.

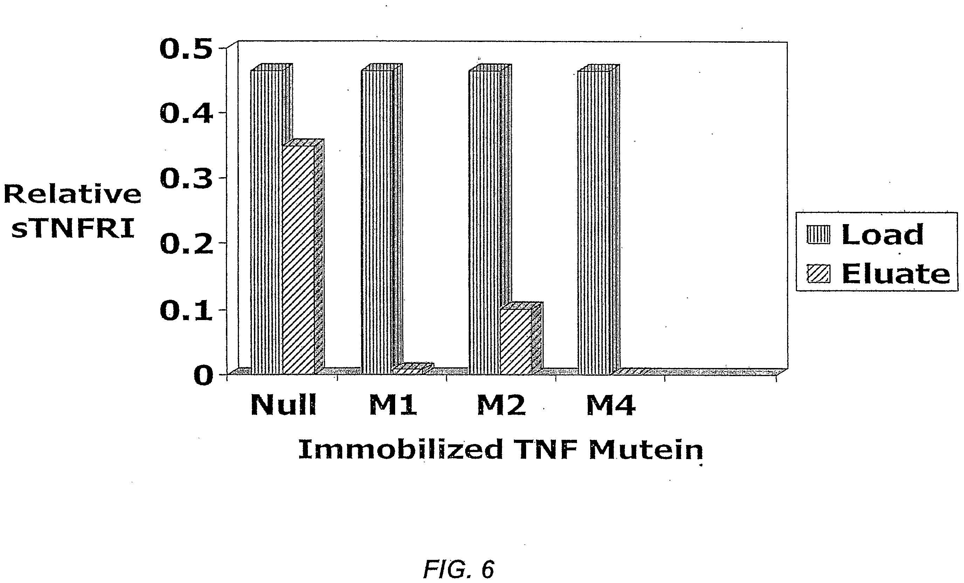

Figure 6 shows the depletion of soluble TNF receptor I (sTNFRI) by immobilized TNF muteins. Muteins 1, 2 and 4 were immobilized on Sepharose™ 4B, and normal human plasma spiked with recombinant human sTNFRI was passed through columns of the immobilized muteins. Depletion of sTNFRI from the plasma was measured by enzyme-linked immunosorbent assay (ELISA).

### DETAILED DESCRIPTION OF THE INVENTION

The present invention may be understood more readily by reference to the following detailed description of preferred aspects of the invention and the Examples included therein and to the Figures and their previous and following description.

Before the present compounds, compositions, articles, devices, and/or methods are disclosed and described, it is to be understood that this invention is not limited to specific synthetic methods, specific nucleic acid molecules, or to particular laser wavelengths, as such may, of course, vary. It is also to be understood that the terminology used herein is for the purpose of describing particular aspects only and is not intended to be limiting.

As used in the specification and the appended claims, the singular forms "a," "an" and "the" include plural referents unless the context clearly dictates otherwise. Thus, for example, reference to a ribonucleic acid includes mixtures of ribonucleic acid molecules, reference to a probe includes mixtures of two or more such probes, and the like.

Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

In this specification and in the claims which follow, reference will be made to a number of terms which shall be defined to have the following meanings:

"Optional" or "optionally" means that the subsequently described event or circumstance may or may not occur, and that the description includes instances where said event or circumstance occurs and instances where it does not. For example, the phrase "the sample optionally may contain more than one TNFα mutein" means that the sample may or may not contain more than one TNFα mutein and that the description includes both a sample containing one TNFα mutein and a sample containing more than one TNFα mutein.

Provided are methods to reduce the levels of immune system inhibitors such as soluble TNF receptors in the circulation of a host mammal, thereby potentiating an immune response capable of resolving a pathological condition or decreasing the severity of a pathological condition. By enhancing the magnitude of the host's immune response, the disclosed methods avoid the problems associated with the repeated administration of chemotherapeutic agents which often have undesirable side effects, for example, chemotherapeutic agents used in treating cancer.

The disclosed methods generally are accomplished by: (a) obtaining a biological fluid from a mammal having a pathological condition; (b) contacting the biological fluid with a TNFα mutein binding partner capable of selectively binding to a targeted immune system inhibitor such as soluble TNF receptor to produce an altered biological fluid having a reduced amount of the targeted immune system inhibitor; and, thereafter (c) administering the altered biological fluid to the mammal.

As used herein, the term "immune system stimulator" refers to soluble mediators that increase the magnitude of an immune response, or which encourage the development of particular immune mechanisms that are more effective in resolving a specific pathological condition. Examples of immune system stimulators include, but are not limited to, the proinflammatory mediators tumor necrosis factors α and *β*, interleukin-1, interleukin-2, interleukin-4, interleukin-5, interleukin-6, interleukin-8, interleukin-12, interferon-γ, interferon-7; and the chemokines RANTES, macrophage inflammatory proteins 1-α and 1-*β*, and macrophage chemotactic and activating factor, as discussed above.

As used herein, the term "immune system inhibitor" refers to a soluble mediator that decreases the magnitude of an immune response, or which discourages the development of particular immune mechanisms that are more effective in resolving a specific pathological condition, or which encourages the development of particular immune mechanisms that are less effective in resolving a specific pathological condition. Examples of host-derived immune system inhibitors include interleukin-1 receptor antagonist, transforming growth factor-*β*, interleukin-4, interleukin-10, or the soluble receptors for interleukin-1, interleukin-2, interleukin-4, interleukin-6, interleukin-7, interferon-γ and tumor necrosis factors α and *β*. In a particular aspect of the disclosed compositions, conjugates and methods, the immune system inhibitor can be soluble TNF receptor Type I (sTNFRI) or Type II (sTNFRII). Immune system inhibitors produced by microorganisms are also potential targets including, for example, soluble receptors for tumor necrosis factor α and *β*. As used herein, the term "targeted" immune system inhibitor refers to that inhibitor, or collection of inhibitors, which is to be removed from the biological fluid by the disclosed methods, for example, sTNFRI and/or sTNFRII.

As used herein, the term "soluble TNF receptor" refers to a soluble form of a receptor for TNFα and TNFβ. Two forms of TNF receptor have been identified, type I receptor (TNFRI), also known as TNF-R55, and type II receptor (TNFRII), also known as TNF-R75, both of which are membrane proteins that bind to TNFα and TNFβ and mediate intracellular signaling. Both of these receptors also occur in a soluble form. The soluble form of TNF receptor functions as an immune system inhibitor, as discussed above. As used herein, a soluble TNF receptor includes at least one of the soluble forms of TNFRI and TNFRII or any other type of TNF receptor. It is understood that, in the disclosed methods, the methods can be used to remove one or both types of TNF receptor depending on whether the TNFα mutein or plurality of muteins, used in the method bind to one or both types of receptors.

As used herein, the term "mammal." can be a human or a non-human animal, such as dog, cat, horse, cattle, pig, sheep, non-human primate, mouse, rat, rabbit, or other mammals, for example. The term "patient" is used synonymously with the term "mammal" in describing the disclosed compositions, conjugates and methods.

As used herein, the term "pathological condition" refers to any condition where the persistence within a host of an agent, immunologically distinct from the host, is a component of or contributes to a disease state. Examples of such pathological conditions include, but are not limited to, those resulting from persistent viral, bacterial, parasitic, and fungal infections, and cancer. Among individuals exhibiting such chronic diseases, those in whom the levels of immune system inhibitors are elevated are particularly suitable for the disclosed treatment. Plasma levels of immune system inhibitors can be determined using methods well known in the art (see, for example, Adolf and Apfler, *supra,* 1991). Those skilled in the art readily can determine pathological conditions that would benefit from the depletion of immune system inhibitors according to the present methods.

As used herein, the term "biological fluid" refers to a bodily fluid obtained from a mammal, for example, blood, including whole blood, plasma, serum, lymphatic fluid, or other types of bodily fluids. If desired, the biological fluid can be processed or fractionated, for example, to obtain an acellular component. As it relates to the disclosed compositions, conjugates and methods, the term "acellular biological fluid" refers to the acellular component of the circulatory system including plasma, serum, lymphatic fluid, or fractions thereof. The biological fluids can be removed from the mammal by any means or methods known to those skilled in the art, including, for example, conventional apheresis methods (see, Apheresis: Principles and Practice, McLeod, Price, and Drew, eds., AABB Press, Bethesda, MD (1997)). The amount of biological fluid to be extracted from a mammal at a given time will depend on a number of factors, including the age and weight of the host mammal and the volume required to achieve therapeutic benefit. As an initial guideline, one plasma volume (approximately 3-5 liters in an adult human) can be removed and, thereafter, depleted of the targeted immune system inhibitor according to the present methods.

As used herein, the term "selectively binds" means that a molecule binds to one type of target molecule, but not substantially to other types of molecules. The term "specifically binds" is used interchangeably herein with "selectively binds."

As used herein, the term "binding partner" is intended to include any molecule chosen for its ability to selectively bind to the targeted immune system inhibitor. The binding partner can be one which naturally binds the targeted immune system inhibitor. For example, tumor necrosis factor α or *β* can be used as a binding partner for sTNFRI. Alternatively, other binding partners, chosen for their ability to selectively bind to the targeted immune system inhibitor, can be used. These include fragments of the natural binding partner, polyclonal or monoclonal antibody preparations or fragments thereof, or synthetic peptides. In a further aspect, the binding partner can be a TNFα mutein which can be a trimer, a dimer, or a monomer.

As used herein, the term "TNFα mutein" refers to a TNFα variant having one or more amino acid substitutions relative to a parent sequence and retaining specific binding activity for a TNF receptor, either soluble and/or membrane TNFR. The mutein of the present invention have a single amino acid substitution relative to a parent sequence. Human TNFα muteins designated muteins 1, 2, 3, 4, 5 and 6 (see Figure 3B), are disclosed which are derived from human TNFα but have a single amino acid substitution relative to the wild type sequence, as discussed below. It is understood that analogous muteins of species other than human are similarly included, for example, muteins analogous to muteins 1, 2, 3, 4, 5 or 6 in the other mammalian species shown in Figure 3A, or other mammalian species. These and other muteins, as described in more detail below, are included within the meaning of a TNFα mutein. Further, TNFα muteins can include mutant or altered forms of TNFα that, for example, have reduced ability to form multimers or are coupled to form a dimer. The disclosed alterations of native TNFα can be used separately or together in any combination. Further, the alterations in muteins 1, 2, 3, 4, 5 and 6 can be used together or separately, and each or all can be used together with alterations that result in monomer or dimer formation. For example, an altered monomer of wild-type TNFα can be coupled with a second altered monomer of wild-type TNFα to form a dimer. In a further aspect, an altered monomer of wild-type TNFα can be coupled with an amino acid sequence, for example mutein 1, to form a dimer. Thus, "TNFα muteins" can include an altered monomer of TNFα; a monomer comprising a single mutation in wild-type TNFα, for example mutein 1, mutein 2, mutein 3, mutein 4, mutein 5, mutein 6, or any other such mutated amino acid sequence described herein; a dimer comprising two identical amino acid sequences; and a dimer containing two non-identical amino acid sequences. For example, a TNFα mutein can include a dimer comprising two identical altered monomers of wild-type TNFα or two non-identical altered monomers of wild-type TNFα. In a further aspect, a TNFα mutein can include a dimer comprising two amino acid sequences that contain an identical amino acid mutation. In yet a further aspect, a TNFα mutein can include a dimer comprising two amino acid sequences that have non-identical amino acid mutations. Further, a TNFα mutein can include a dimer comprising an amino acid sequence of an altered form of wild-type TNFα and an amino acid sequence containing a single amino acid mutation, for example an amino acid sequence identified by SEQ ID NO:3, SEQ ID NO:4, SEQ ID NO:5, SEQ ID NO:6, SEQ ID NO:7, SEQ ID NO:8, or any other mutated amino acid sequence disclosed herein.

A TNFα mutein can be monomeric, dimeric or trimeric. That is, a TNFα mutein can be in a monomeric form, a dimeric form, or a trimeric form. Native TNFα exists as a homotrimer of three 17 kDa subunits, as do the TNFα muteins exemplified herein. Other TNFα muteins produced using the methods described herein also can exist as trimers. Binding of TNFα or TNFα muteins to the monovalent soluble TNFR involves only two of the three monomers present in the TNFα or TNFα mutein trimer. While the TNFα or TNFα mutein trimer can bind to three monovalent soluble TNFR molecules, the binding to each is an independent event which is governed solely by affinity and which does not allow for a contribution by avidity. Therefore, the strength of binding by a dimeric form of TNFα or TNFα mutein to soluble TNFR is undiminished relative to the trimeric form of TNFα or TNFα muteins.

Provided are compositions and methods for stimulating or enhancing an immune response in a mammal. The invention uses ligands that as defined in the claims, bind to immune system inhibitors to counterbalance or decrease the dampening effect of immune system inhibitors on the immune response. Such ligands, can be attached to a solid support to allow the removal of an immune system inhibitor from a biological fluid.

A binding partner particularly useful in the present invention is a ligand that binds with high affinity to a soluble TNF receptor and in particular sTNFRI and/or sTNFRII. Another useful characteristic of a binding partner is a lack of direct toxicity. For example, a binding partner lacking or having reduced TNF agonist activity is particularly useful. Generally, even when a ligand such as a binding partner is covalently bound to a solid support, a certain percentage of the bound ligand will leach from the support, for example, via chemical reactions that break down the covalent linkage or protease activity present in a biological fluid. In such a case, the ligand will leach into the biological fluid being processed and, thus, be returned to the patient. Therefore, it is advantageous to use a ligand that has affinity for an immune system inhibitor but has decreased ability to stimulate a biological response, that is, has decreased or low agonist activity. In this case, even if some of the ligand leaches into the processed biological fluid, the ligand would still exhibit low biological activity with respect to membrane receptor signaling when reintroduced into the patient.

Yet another useful characteristic of a binding partner is a lack of indirect toxicity, for example, immunogenicity. As discussed above, it is common for a bound ligand to leach from a matrix, resulting in the ligand being present in the processed biological fluid. When the biological fluid is returned to the patient, this results in the introduction of a low level of the ligand to the patient. If the ligand is immunogenic, an immune response against the ligand can be stimulated, resulting in undesirable immune responses, particularly in a patient in which the process is being repeated. Therefore, a ligand having low immunogenicity would minimize any undesirable immune responses against the ligand. As disclosed herein, a particularly useful ligand to be used as a binding partner of the invention is derived from the same species as the patient being treated. For example, for treating a human, a human TNFα mutein can be used as the binding partner, which is expected to have low immunogenicity given the homology to the endogenous TNFα. Similarly, muteins derived from other mammalian species can be used in the respective species.

As disclosed herein, TNFα muteins are particularly useful binding partners in methods of the invention. A number of TNFα muteins have been previously described (see, for example, Van Ostade et al., Protein Eng. 7:5-22 (1994); Van Ostade et al., EBMO J. 10:827-836 (1991); Zhang et al., J. Biol. Chem. 267:24069-24075 (1992); Yamagishi et al., Protein Eng. 3:713-719 (1990)). Specific exemplary muteins include the human TNFα muteins shown in Figure 3B.

There are several advantages to using TNFα muteins as binding partners in the present invention. Although TNFα muteins can display lower binding activity for TNF receptors, some TNFα muteins bind only 5- to 17-fold less effectively than native TNFα. Such a binding affinity, albeit reduced relative to native TNFα, can still be an effective binding partner in the present invention (see Example 3). Another advantage of using TNFα muteins is that some exhibit decreased signaling through membrane receptors, for example, decreased cytotoxic activity or *in vivo* toxicity, relative to native TNFα. In particular, muteins 1, 2, 3, 4, 5 and 6 exhibit a 200- to 10,000-fold decrease in cytotoxicity (see below and Van Ostade, *supra,* 1994; Yamagishi et al., *supra,* 1990; Zhang et al., *supra,* 1992). Thus, even though the binding affinity is reduced 10- to 17-fold, there can be a 200- to 10,000-fold decrease in signaling through membrane receptors, for example, decreased cytotoxic activity or *in vivo* toxicity. As discussed above, such a reduced signaling through membrane receptors, for example, reduced cytotoxicity or *in vivo* toxicity, is advantageous in view of the potential leaching of the ligand from a matrix and introduction of low levels into a patient when an altered biological fluid is returned to the patient. Further, dimeric fusion proteins of TNFα or dimeric forms of TNFα muteins can be used in the disclosed methods. Such dimeric fusions are useful because (1) they bind soluble TNFR receptor with affinities sufficient to remove the soluble TNFR from a biological fluid and (2) because they display reduced binding to, or signaling through, membrane TNFR relative to wild type TNFα, thus reducing or eliminating toxicity.

An additional advantage of using TNFα muteins is that they have a native structure. Because the muteins are highly homologous to the native TNFα sequence, these muteins can fold into a native structure that retains TNF receptor binding activity. Such a native structure means that the same amino acid residues are exposed on the surface of the molecule as in the native TNFα, except for possibly the mutant amino acid residue. Such a native folding means that the TNFα muteins should have little or no immunogenicity in the respective mammalian species.

As disclosed herein, particularly useful muteins are human muteins 1, 2, 3, 4, 5 and 6 (Figure 3B) and the analogous muteins in other mammalian species. Mutein 1 is a single amino acid substitution relative to wild type human TNFα of Arg³¹ with Pro (Zhang et al., *supra,* 1992). This mutein exhibits approximately 10-fold lower binding activity to membrane TNFR and approximately 10,000-fold lower cytotoxicity relative to native TNFα. Mutein 2 is a single amino acid substitution relative to wild type human TNFα of Asn³⁴ with Tyr (Yamagishi et al., *supra,* 1990; Asn³² in the numbering system of Yamagishi et al.). This mutein exhibits approximately 5-fold lower binding activity to membrane TNFR and approximately 12,500-fold lower cytotoxicity relative to native TNFα. Mutein 3 is a single amino acid substitution relative to wild type human TNFα of Pro¹¹⁷ with Leu (Yamagishi et al., *supra,* 1990; Pro 115 in the numbering system of Yamagishi et al.). This mutein exhibits approximately 12-fold lower binding activity to membrane TNFR and approximately 1400-fold lower cytotoxicity. Mutein 4 is a single amino acid substitution relative to wild type human TNFα of Ser¹⁴⁷ with Tyr (Zhang et al., *supra,* 1992). This mutein exhibits approximately 14-fold lower binding activity to membrane TNFR and approximately 10,000-fold lower cytotoxicity relative to native TNFα. Mutein 5 is a single amino acid substitution relative to wild type human TNFα of Ser⁹⁵ with Tyr (Zhang et al., *supra,* 1992). This mutein exhibits approximately 17-fold lower binding activity to membrane TNFR and approximately 200-fold lower cytotoxicity relative to native TNFα. Mutein 6 is a single amino acid substitution relative to wild type human TNFα of Tyr¹¹⁵ with Phe (Zhang et al., *supra*, 1992). This mutein exhibits approximately 17-fold lower binding activity to membrane TNFR and approximately 3,300-fold lower cytotoxicity relative to native TNFα. As disclosed herein, it is understood that analogous muteins can be generated in other mammalian species by making the same amino acid substitutions in the analogous position of the respective species.

Although muteins 1, 2 and 4, as well as other TNFα muteins, were previously known and characterized with respect to binding the multivalent membrane receptor, it was previously unknown whether these TNFα muteins would bind to the monovalent soluble TNF receptors. As disclosed herein, the TNFα muteins bind with an affinity sufficient to deplete soluble TNF receptor from plasma (see Examples 3 and 6). These results indicate that TNFα muteins can be an effective binding partner for depleting soluble TNF receptor from a biological fluid.

TNFα from various mammalian species show a high degree of amino acid identity (see Figure 3A and 3B, conserved sequence SEQ ID NO:1; Van Ostade et al., *supra,* 1994). As described by Van Ostade et al. (*supra*, 1994), a conserved TNFα amino acid sequence was identified across 11 mammalian species. The conserved amino acid residues are conserved across all 11 shown species or have only a single species showing variation at that position (see Figure 3A and Van Ostade et al., *supra,* 1994). Thus, provided is a TNFα mutein comprising the conserved sequence referenced as SEQ ID NO:1.

One skilled in the art can readily determine additional muteins suitable for use in the disclosed compositions, conjugates and methods. As discussed above, TNFα muteins having relatively high affinity for TNF receptors and decreased signaling through membrane receptors, for example, decreased cytotoxicity or *in vivo* toxicity, relative to native TNFα are particularly useful in the disclosed compositions, conjugates and methods. One skilled in the art can readily determine additional suitable TNFα muteins based on methods well known to those skilled in the art. Methods for introducing amino acid substitutions into a sequence are well known to those skilled in the art (Ausubel et al., Current Protocols in Molecular Biology (Supplement 56), John Wiley & Sons, New York (2001); Sambrook and Russel, Molecular Cloning: A Laboratory Manual, 3rd ed., Cold Spring Harbor Press, Cold Spring Harbor (2001); U.S. Patent Nos. 5,264,563 and 5,523,388). Generation of TNFα muteins has been previously described (Van Ostade et al., *supra,* 1994; Van Ostade et al., *supra,* 1991; Zhang et al., *supra,* 1992; Yamagishi et al., *supra,* 1990). Furthermore, one skilled in the art can readily determine the binding and cytotoxicity and/or *in vivo* toxicity of candidate muteins to ascertain the suitability for use in the disclosed method (Van Ostade et al., *supra*, 1994; Van Ostade et al., *supra,* 1991; Zhang et al., *supra,* 1992; Yamagishi et al., *supra,* 1990).

TNFα muteins of particular interest for use in the disclosed compositions, conjugates and methods, in addition to having relatively high affinity for TNF receptors and reduced signaling through membrane receptors, for example, reduced cytotoxicity or *in vivo* toxicity, are those having amino acid substitutions in three regions of TNFα: region 1, amino acids 29-36; region 2, amino acids 84-91; and region 3, amino acids 143-149 (numbering as shown in Figure 3A). Muteins 1, 2 and 4 are exemplary of muteins having single amino acid substitutions in these regions. Region 1 corresponds to amino acids 29-36, residues LNRRANAL (amino acids 29-36 of SEQ ID NO:2) of human TNFα. Region 2 corresponds to amino acids 84-91, residues AVSYQTKV (amino acids 84-91 of SEQ ID NO:2) of human TNFα. Region 3 corresponds to amino acids 143-149, residues DFAESG (SEQ ID NO:20) of human TNFα. In addition to the TNFα muteins disclosed herein, other TNFα mutein can be generated, for example, by introducing single amino acid substitutions in regions 1,2 or 3 and screening for binding activity and cytotoxic activity and/or *in vivo* toxicity as disclosed herein (see also Van Ostade et al., *supra,* 1991; Zhang et al, *supra,* 1992; Yamagishi et al., *supra*, 1990). Methods for introducing amino acid substitutions at a particular amino acid residue or region are well known to those skilled in the art (see, for example, Van Ostade et al., *supra*, 1991; Zhang et al, *supra,* 1992; Yamagishi et al., *supra,* 1990; U.S. Patent Nos. 5,264,563 and 5,523,388). For example, each of the other 19 amino acids relative to a native sequence can be introduced at each of the positions in regions 1, 2 and 3 and screened for binding activity and/or signaling activity, for example, cytotoxic activity or *in vivo* toxicity, to soluble and/or membrane bound TNF receptor. This would only require the generation of approximately 420 mutants (19 single amino acid substitutions at each of 22 positions in regions 1, 2 and 3), a number which can be readily generated and screened by well known methods. Those having desired characteristics as disclosed herein, for example, specific binding activity for soluble TNF receptor and reduced signaling through the membrane TNF receptor, can be selected as a TNFα mutein useful in the disclosed compositions, conjugates and methods.

Also disclosed is a TNFα mutein having the consensus sequence of SEQ ID NO:9 (Figure 3C). In one aspect, a TNFα mutein comprises the consensus sequence SEQ ID NO:9, wherein X₁ is an amino acid selected from Leu and Val; wherein X₂ is a 2 or 3 amino acid peptide having Gln or Arg at position 1, Asn, Ala or Thr at position 2, and Ser, Leu, Pro or absent at position 3, for example, selected from GlnAsnSer, ArgAlaLeu, ArgThrPro, GlnAlaSer, and GlnThr; wherein X₃ is an amino acid selected from Asp and Asn; wherein X₄ is a 5 amino acid peptide having His, Pro, Leu, Ile or Val at position 1, Gln, Glu, Ser, Asn or Lys at position 2, Val, Ala or Ser at position 3, Glu or Pro at position 4, and Glu or Gly at position 5, for example, selected from HisGlnValGluGlu (SEQ ID NO:21), HisGlnAlaGluGlu (SEQ ID NO:22), ProGlnValGluGly (SEQ ID NO:23), ProGluAlaGluGly (SEQ ID NO:24), LeuSerAlaProGly (SEQ ID NO:25), IleSerAlaProGly (SEQ ID NO:26), ProGlnAlaGluGly (SEQ ID NO:27), IleAsnSerProGly (SEQ ID NO:28), and ValLysAlaGluGly (SEQ ID NO:29); wherein X₅ is an amino acid selected from Glu, Gln and Arg; wherein X₆ is a 4 amino acid peptide having Leu, Gly, Trp or Gln at position 1, Ser, Asp or Asn at position 2, Gln, Arg, Ser or Gly at position 3, and Arg or Tyr at position 4, for example, selected from LeuSerGlnArg (SEQ ID NO:30), LeuSerArgArg (SEQ ID NO:31), GlyAspSerTyr (SEQ ID NO:32), LeuSerGlyArg (SEQ ID NO:33), TrpAspSerTyr (SEQ ID NO:34), GlnSerGlyTyr (SEQ ID NO:35), and LeuAsnArgArg (SEQ ID NO:36); wherein X₇ is an amino acid selected from Leu, Met, and Lys; wherein X₈ is a two amino acid peptide having Met or Val at position 1 and Asp, Lys, Glu or Gln at position 2, for example, selected from MetAsp, MetLys, ValGlu, ValLys, and ValGln; wherein X₉ is an amino acid selected from Lys, Thr, Glu, and Arg; wherein X₁₀ is an amino acid selected from Val, Lys, and Ile; wherein X₁₁ is a 2 amino acid peptide having Ala, Ser, Thr or Leu at position 1 and Asp or Glu at position 2, for example, selected from AlaAsp, SerAsp, ThrAsp, LeuAsp, AlaGlu, and SerGlu; wherein X₁₂ is an amino acid selected from Lys, Ser, Thr, and Arg; wherein X₁₃ is an amino acid selected from Gln and His; wherein X₁₄ is a 4 or 5 amino acid peptide having Asp, Ser or Pro at position 1, Val, Tyr, Pro or Thr at position 2, Val, Pro, His or Asn at position 3, Leu or Val at position 4, and Leu, Phe or absent at position 5, for example, selected from AspValValLeu (SEQ ID NO:37), AspTyrValLeu (SEQ ID NO:38), SerTyrValLeu (SEQ ID NO:39), ProProProVal (SEQ ID NO:40), SerThrHisValLeu (SEQ ID NO:41), SerThrProLeuPhe (SEQ ID NO:42), and SerThrAsnValPhe (SEQ ID NO:43); wherein X₁₅ is an amino acid selected from Val and Ile; wherein X₁₆ is an amino acid selected from Phe, Ile, and Leu; wherein X₁₇ is an amino acid selected from Ile and Val; wherein X₁₈ is a 2 amino acid peptide having Gln or Pro at position 1 and Glu, Asn, Thr or Ser at position 2, for example, selected from GlnGlu, ProAsn, GlnThr, and ProSer; wherein X₁₉ is an amino acid selected from Leu and Ile; wherein X₂₀ is a 3 amino acid peptide having Pro, His or Gln at position 1, Lys, Arg or Thr at position 2, and Asp or Glu at position 3, for example, selected from ProLysAsp, HisArgGlu, GlnArgGlu, and HisThrGlu; wherein X₂₁ is an amino acid selected from Gly, Glu, Gln, and Trp or is absent; wherein X₂₂ is an amino acid selected from Leu, Pro, and Ala; wherein X₂₃ is an amino acid selected from Leu and Gln; wherein X₂₄ is an amino acid selected from Gly and Asp; wherein X₂₅ is an amino acid selected from Gln, Leu, and Arg; wherein X₂₆ is an amino acid selected from Ala and Thr; wherein X₂₇ is an amino acid selected from Val and Ile; wherein X₂₈ is an amino acid selected from Leu, Gln, and Arg; wherein X₂₉ is an amino acid selected from Lys, Glu, Ala, Asn, and Asp; wherein X₃₀ is an amino acid selected from Phe, Ile, Leu and Tyr; and wherein X₃₁ is an amino acid selected from Val and Ile (see Figure 3A; Van Ostade et al., *supra,* 1994). Such a consensus TNFα mutein is expected to exhibit binding activity for TNF receptor, and such activity can be readily determined by those skilled in the art using well known methods, as disclosed herein.

In addition to the variant positions described above, it is understood that a TNFα mutein can additionally include variant amino acids in the conserved sequence referenced as SEQ ID NO:1. As shown in Figure 3A and as discussed above, the conserved TNFα sequence includes certain positions where one of the shown mammalian species differs from the other ten. For example, the conserved amino acid at position 2, Arg, is Leu in dog (Figure 3A). Thus, a TNFα mutein can include a substitution of Leu at position 2 with the remainder of the conserved sequence referenced as SEQ ID NO:1. Similarly, substitutions of other "conserved" positions, where at least one of the species has an amino acid substitution relative to the conserved sequence, are included as TNFα muteins. For example, a TNFα mutein can have the corresponding substitution of mutein 1, that is, Arg³¹Pro and substitution in the conserved sequence in the variable positions, as described above represented by X, and/or substitution in a conserved position that varies in a single species. Furthermore, a TNFα mutein can include conservative amino acid substitutions relative to the conserved sequence or the sequence of a particular species of TNFα. Such TNFα muteins can be readily recognized by one skilled in the art based on the desired characteristics of a TNFα mutein, as disclosed herein.

Any of the TNFα muteins disclosed herein can be modified to include an N-terminal deletion. As discussed in Van Ostade (*supra*, 1994), short deletions at the N-terminus of TNFα retained activity, whereas deletion of the N-terminal 17 amino acids resulted in a loss of activity. Therefore, it is understood that the disclosed TNFα muteins also include TNFα muteins having N-terminal deletions that retain activity. Such TNFα muteins can include, for example, an N-terminal deletion of 1, 2, 3, 4, 5, 6, 7, 8, 9 or 10 amino acids. Furthermore, one skilled in the art can readily determine whether further N-terminal deletions can be incorporated into a TNFα muteins by making the deletion mutations and screening for desired characteristics, as disclosed herein.

Provided are a variety of TNFα muteins as disclosed herein. Generally, a particularly useful TNFα mutein has about 2-fold, about 3-fold, about 4-fold, about 5-fold, about 6-fold, about 7-fold, about 8-fold, about 9-fold, about 10-fold, about 11-fold, about 12-fold, about 13-fold, about 14-fold, about 15-fold, about 16-fold, about 17-fold, about 18-fold, about 19-fold, about 20-fold, about 25-fold, about 30-fold, or even higher fold reduced binding affinity for TNF receptors, particularly membrane bound TNF receptors, relative to native/wild type TNFα. Such reduced binding affinity can be, but is not necessarily, exhibited toward sTNFR. Also, a particularly useful TNFα mutein has about 5-fold, about 10-fold, about 50-fold, about 100-fold, about 150-fold, about 200-fold, about 300-fold, about 500-fold, about 1000-fold, about 2000-fold, about 3000-fold, about 4000-fold, about 5000-fold, about 6000-fold, about 7000-fold, about 8000-fold, about 9000-fold, about 10,000-fold, about 20,000-fold, about 30,000-fold, about 50,000-fold, or even higher fold reduced signaling through the membrane receptors, for example, reduced cytoxicity or *in vivo* toxicity, relative to native/wild type TNFα. It is understood that a TNFα mutein can have reduced binding affinity and/or reduced cytoxicity, as discussed above and disclosed herein.

Provided is a conjugate comprising a tumor necrosis factor α (TNFα) mutein attached to a substrate. In one further aspect, the TNFα mutein of the conjugate comprises the conserved sequence referenced as SEQ ID NO:1.

Further disclosed is a conjugate where the TNFα mutein has the consensus sequence SEQ ID NO:9, wherein X₁ is an amino acid selected from Leu and Val; wherein X₂ is a 2 or 3 amino acid peptide having Gln or Arg at position 1, Asn, Ala or Thr at position 2, and Ser, Leu, Pro or absent at position 3, for example, selected from GlnAsnSer, ArgAlaLeu, ArgThrPro, GlnAlaSer, and GlnThr; wherein X₃ is an amino acid selected from Asp and Asn; wherein X₄ is a 5 amino acid peptide having His, Pro, Leu, Ile or Val at position 1, Gln, Glu, Ser, Asn or Lys at position 2, Val, Ala or Ser at position 3, Glu or Pro at position 4, and Glu or Gly at position 5, for example, selected from HisGlnValGluGlu (SEQ ID NO:21), HisGlnAlaGluGlu (SEQ ID NO:22), ProGlnValGluGly (SEQ ID NO:23), ProGluAlaGluGly (SEQ ID NO:24), LeuSerAlaProGly (SEQ ID NO:25), IleSerAlaProGly (SEQ ID NO:26), ProGlnAlaGluGly (SEQ ID NO:27), IleAsnSerProGly (SEQ ID NO:28), and ValLysAlaGluGly (SEQ ID NO:29); wherein X₅ is an amino acid selected from Glu, Gln and Arg; wherein X₆ is a 4 amino acid peptide having Leu, Gly, Trp or Gln at position 1, Ser, Asp or Asn at position 2, Gln, Arg, Ser or Gly at position 3, and Arg or Tyr at position 4, for example, selected from LeuSerGlnArg (SEQ ID NO:30), LeuSerArgArg (SEQ ID NO:31), GlyAspSerTyr (SEQ ID NO:32), LeuSerGlyArg (SEQ ID NO:33), TrpAspSerTyr (SEQ ID NO:34), GlnSerGlyTyr (SEQ ID NO:35), and LeuAsnArgArg (SEQ ID NO:36); wherein X₇ is an amino acid selected from Leu, Met, and Lys; wherein X₈ is a two amino acid peptide having Met or Val at position 1 and Asp, Lys, Glu or Gln at position 2, for example, selected from MetAsp, MetLys, ValGlu, ValLys, and ValGln; wherein X₉ is an amino acid selected from Lys, Thr, Glu, and Arg; wherein X₁₀ is an amino acid selected from Val, Lys, and Ile; wherein X₁₁ is a 2 amino acid peptide having Ala, Ser, Thr or Leu at position 1 and Asp or Glu at position 2, for example, selected from AlaAsp, SerAsp, ThrAsp, LeuAsp, AlaGlu, and SerGlu; wherein X₁₂ is an amino acid selected from Lys, Ser, Thr, and Arg; wherein X₁₃ is an amino acid selected from Gln and His; wherein X₁₄ is a 4 or 5 amino acid peptide having Asp, Ser or Pro at position 1, Val, Tyr, Pro or Thr at position 2, Val, Pro, His or Asn at position 3, Leu or Val at position 4, and Leu, Phe or absent at position 5, for example, selected from AspValValLeu (SEQ ID NO:37), AspTyrValLeu (SEQ ID NO:38), SerTyrValLeu (SEQ ID NO:39), ProProProVal (SEQ ID NO:40), SerThrHisValLeu (SEQ ID NO:41), SerThrProLeuPhe (SEQ ID NO:42), and SerThrAsnValPhe (SEQ ID NO:43); wherein X₁₅ is an amino acid selected from Val and Ile; wherein X₁₆ is an amino acid selected from Phe, Ile, and Leu; wherein X₁₇ is an amino acid selected from Ile and Val; wherein X₁₈ is a 2 amino acid peptide having Gln or Pro at position 1 and Glu, Asn, Thr or Ser at position 2, for example, selected from GlnGlu, ProAsn, GlnThr, and ProSer; wherein X₁₉ is an amino acid selected from Leu and Ile; wherein X₂₀ is a 3 amino acid peptide having Pro, His or Gln at position 1, Lys, Arg or Thr at position 2, and Asp or Glu at position 3, for example, selected from ProLysAsp, HisArgGlu, GlnArgGlu, and HisThrGlu; wherein X₂₁ is an amino acid selected from Gly, Glu, Gln, and Trp or is absent; wherein X₂₂ is an amino acid selected from Leu, Pro, and Ala; wherein X₂₃ is an amino acid selected from Leu and Gln; wherein X₂₄ is an amino acid selected from Gly and Asp; wherein X₂₅ is an amino acid selected from Gln, Leu, and Arg; wherein X₂₆ is an amino acid selected from Ala and Thr; wherein X₂₇ is an (amino acid selected from Val and Ile; wherein X₂₈ is an amino acid selected from Leu, Gln, and Arg; wherein X₂₉ is an amino acid selected from Lys, Glu, Ala, Asn, and Asp; wherein X₃₀ is an amino acid selected from Phe, Ile, Leu and Tyr; and wherein X₃₁ is an amino acid selected from Val and He.

In a further aspect, provided is a conjugate where the TNFα mutein has an amino acid substitution in a region of TNFα selected from region 1 amino acids 29-36, region 2 amino acids 84-91 and region 3 amino acids 143-149 of human TNFα (SEQ ID NO:2) or the analogous position of TNFα from another species.

Also, disclosed is a conjugate where the TNFα mutein is selected from mutein 1 (SEQ ID NO:3), mutein 2 (SEQ ID NO:4), mutein 3 (SEQ ID NO:5), mutein 4 (SEQ ID NO:6), mutein 5 (SEQ ID NO:7) and mutein 6 (SEQ ID NO:8). In a particular aspect, provided is a conjugate where the TNFα mutein is selected from mutein 1 (SEQ ID NO:3), mutein 2 (SEQ ID NO:4), and mutein 4 (SEQ ID NO:6). In the dimeric form, a TNFα mutein can comprise two identical amino acid sequences, or two non-identical amino acid sequences. The TNFα mutein of the conjugate can be derived from a species selected, for example, from human, dog, cat, horse, sheep, goat, pig, cow, rabbit and rat.

Further disclosed is a method of stimulating an immune response in a mammal having a pathological condition. The method can include the steps of obtaining a biological fluid from the mammal; contacting the biological fluid with a tumor necrosis factor α (TNFα) mutein having specific binding activity for a soluble tumor necrosis factor receptor (TNFR); removing the TNFα mutein bound to the soluble TNFR from the biological fluid to produce an altered biological fluid having a reduced amount of soluble TNFR; and administering the altered biological fluid to the mammal. The biological fluid can be, for example, blood, plasma, serum or lymphatic fluid, including whole blood. In a further aspect, a method using whole blood as the biological fluid can further include the step of separating the whole blood into a cellular component and an acellular component or a fraction of the acellular component, wherein the acellular or the fraction of the acellular component contains a soluble TNFR. The method can additionally include the step of combining the cellular component with the altered acellular component or altered fraction of the acellular component to produce altered whole blood, which can be administered to the mammal as the altered biological fluid. Accordingly, the cellular component and the altered acellular component or altered fraction of the acellular component can be administered separately to the mammal.

A TNFα mutein can have specific binding activity for a single type of soluble TNFR, for example sTNFRI or sTNFRII. Alternatively, the TNFα mutein can have specific binding activity for more than one type of soluble TNFR, for example, both sTNFRI and sTNFRII.

Various mixtures of binding partners can be used. For example, one mixture can be composed of multiple binding partners that selectively bind to a single targeted immune system inhibitor. Another mixture can be composed of multiple binding partners, each of which selectively binds to different targeted immune system inhibitors. Alternatively, the mixture can be composed of multiple binding partners that selectively bind to different targeted immune system inhibitors. For example, the mixture can contain more than one TNFα mutein. Furthermore, the mutiple TNFα muteins can specifically bind to a single type of soluble TNF receptor or can bind to more than one type of TNF receptor, for example, sTNFRI and sTNFRII.

In a further aspect, the biological fluid can be contacted with a plurality of TNFα muteins. Therefore, the plurality of TNFα muteins can have specific binding activity for a single type of soluble TNFR, for example, sTNFRI or sTNFRII. Alternatively, the plurality of TNFα muteins can have specific binding activity for more than one type of soluble TNFR, that is, sTNFRI and sTNFRII.

When it is desirable to increase the molecular weight of the binding partner/immune system inhibitor complex, the binding partner can be conjugated to a carrier. Examples of such carriers include, but are not limited to, proteins, complex carbohydrates, and synthetic polymers such as polyethylene glycol.

As used herein, "functionally active binding sites" of a binding partner refer to sites that are capable of binding to one or more targeted immune system inhibitors.

One method to generate a dimeric form of a TNFα or TNFα mutein is to produce a fusion protein which covalently links a TNFα or TNFα muteins monomer to an antibody heavy chain constant region. A particularly useful method to generate a dimeric TNFα is to fuse a TNFα to each of the two heavy chain constant regions involved in forming the dimeric Fc portion of an antibody. As these heavy chain constant region monomers assemble to form the stable Fc structure, they will cause the associated TNFα or TNFα mutein molecules to dimerize as well. In one aspect, the two amino acid sequences of the dimeric form are identical; in another aspect, the two amino acid sequences of the dimeric form are non-identical. In yet another aspect, a fusion protein can be produced in which the amino terminus of the TNFα muteins is fused to the carboxy-tenninus of an intact heavy chain constant region. TNFα has been fused at its amino terminus in a variety of fusion proteins without a significant loss of biological activity. Such a heavy chain-TNFα mutein fusion protein can be assembled with intact antibody light chains to form a molecule that would neutralize soluble TNFR, and possess limited immunogenicity in a mammal from whom the antibody and TNFα sequences are derived. In another aspect, the heavy chain constant region can be truncated amino-terminal of the hinge region, thereby providing two sites to which a TNFα or TNFα mutein monomer can be fused at its carboxy terminus. Fusion proteins involving the carboxy terminus of TNFα have been produced, but typically these have resulted in significant losses in the biological activity of the TNFα component of the fusion protein. The observed losses in activity result from the fact that the carboxyl group of the carboxy terminal amino acid of TNFα (Leu¹⁵⁷) forms an ion pair with Lys¹¹ in an adjacent monomer, thus stabilizing trimer formation (Eck and Sprang, J. Biol. Chem. 264(29): 17595-17605 (1989)). If the formation of a peptide bond that utilizes the carboxyl group of Leu¹⁵⁷ leads to diminished binding of the fusion protein for soluble TNFR, one or more amino acids can be inserted into the junction between the amino terminus of the heavy chain constant region and the carboxy terminus of the TNFα mutein, the amino acids having R-groups that contain carboxyl functionalities (for example, Asp or Glu). In yet another aspect, other dimeric plasma proteins, including either homodimers or heterodimers, can be fused to the amino or carboxy termini of a TNFα mutein as described above.

Alternatively, a TNFα monomer can be cross-linked to a plasma protein, for example, an antibody or serum albumin, using well known chemical cross-linking methods. Such methods are well known as taught, for example, in Hermanson, Bioconjugate Techniques, Academic Press, San Diego (1996).

Also provided are momneric forms of TNFα and TNFα muteins, which can be generated to further reduce the binding to, and signaling through, membrane TNFR. The monomeric form of TNFα is anticipated to have the lowest binding strength of any TNFα isoform for membrane TNFR due to the absence of any contribution for avidity. Further, monomeric TNFα is anticipated to signal through membrane TNFR significantly less well than trimeric TNFα since the ability of monomeric TNFα to crosslink membrane TNFR is reduced. As noted above, binding of TNFα or TNFα muteins to the monovalent soluble TNFR involves only two of the three monomers present in the TNFα or TNFα mutein trimer. Thus, TNFα and TNFα muteins must be multimerized to at least a dimeric state in order to bind soluble TNFR. Multimerization of monomeric TNFα or TNFα mutein is readily accomplished by their covalent conjugation to a solid support in creating the adsorbent matrix disclosed herein. Thus, two adjacent molecules of monomeric TNFα or TNFα mutein, once immobilized in proximity to one another on the solid support, can bind soluble TNFR. Upon dissociation from the solid support, however, these ligands will return to the monomeric state and, thus, bind to and signal poorly through membrane TNFR.

One method to generate a monomeric form of a TNFα or TNFα mutein is to produce a fusion protein in which the TNFα or TNFα mutein monomer is fused at its carboxy terminus to amino acids not present in wild type TNF. As noted above, carboxy-terminal fusions of TNFα have been produced, but typically these have resulted in significant losses in the biological activity of the TNFα component of the fusion protein. The observed losses in activity result from the fact that the carboxyl group of the carboxy terminal amino acid of TNFα (Leu¹⁵⁷) forms an ion pair with Lys¹¹ in an adjacent monomer, thus stabilizing trimer formation (Eck and Sprang, J. Biol. Chem. 264(29): 17595-17605 (1989)). Incoiporation of additional amino acids, which extend the carboxy terminus of the molecule a distance sufficient to prevent ion pairing with Lys¹¹ in an adjacent monomer, should decrease trimer formation. Amino acids that may be fused to the carboxy terminus of the TNFα or TNFα mutein may include a purification tag (e.g., polyhistidine or GST) or any random amino acid sequence that allows proper folding of the monomer and which preferably is not immunogenic.

In another aspect, mutations can be introduced into the TNFα or TNFα mutein portion of a dimeric fusion protein, such mutations being designed to reduce the ability of the dimerized TNFα or TNFα mutein to associate with a monomer of wild type TNFα. Association of dimeric TNFα or TNFα mutein with a monomer of wild type TNFα would restore the trimeric structure and potentially increase the ability of the fusion protein to bind to membrane TNFR and, thus, contribute to toxicity, if the fusion protein were to be released from the adsorbent matrix and returned to the patient. The introduction of mutations at residues which normally form ion pairs that contribute to the assembly of trimeric TNFα or TNFα muteins (for example, Lys⁹⁸ and Glu¹¹⁶ or Lys¹¹ and Leu¹⁵⁷, Eck and Sprang, *supra*, 1989) would reduce or eliminate the ability of such muteins to associate with wild type TNFα monomers. As discussed above, fusion of TNFα or TNFα muteins at the carboxy terminal Leu¹⁵⁷ to an immunoglobulin heavy chain or other fusion partner can serve to prevent association with a wild type TNFα monomer by preventing the formation of an ion pair with Lys¹¹ in an adjacent subunit.

Methods for producing the various binding partners useful with the disclosed compositions, conjugates and emthods are well known to those skilled in the art. Such methods include, for example, recombinant DNA and synthetic techniques, or a combination thereof. Binding partners such as TNFα muteins can be expressed in prokaryotic or eukaroytic cells, for example, mammalian, insect, yeast, and the like. If desired, codons can be changed to reflect any codon bias in a host species used for expression.

A binding partner, such as a TNFα mutein, can be attached to an inert medium to form an adsorbent matrix (Figure 1). The TNFα mutein can be, for example, covalently attached to a substrate such as an inert medium. As used herein, the term "inert medium" is intended to include solid supports to which the binding partner(s) can be attached. Particularly useful supports are materials that are used for such purposes including, for example, cellulose-based hollow fibers, synthetic hollow fibers, silica-based particles, flat or pleated membranes, macroporous beads, agarose-based particles, and the like. The inert medium can be in the form of a bead, for example, a macroporous bead or a non-porous bead. Exemplary macroporous beads include, but are not limited to, naturally occurring materials such as agarose, cellulose, controlled pore glass, or synthetic materials such as polyacrylamide, cross-linked agarose (such as Trisacryl™, Sephacryl, Actigel™, and Ultrogel™), azlactone, polymethacrylate, polystyrene/divinylbenzene, and the like. In one aspect, a macroporous bead comprises Actigel™. Exemplary non-porous beads include, but are not limited to, silica, polystyrene, latex, and the like. Hollow fibers and membranes can also be composed of natural or synthetic materials. Exemplary natural materials include, but are not limited to, cellulose and modified cellulose, for example, cellulose diacetate or triacetate. Exemplary synthetic materials include, but are not limited to, polysulfone, polyvinyl, polyacetate, and the like. Such inert media can be obtained commercially or can be readily made by those skilled in the art. The binding partner can be attached to the inert medium by any means or methods known to those skilled in the art including, for example, covalent conjugation. Alternatively, the binding partner can be associated with the inert matrix through high-affinity, noncovalent interaction with an additional molecule which has been covalently attached to the inert medium. For example, a biotinylated binding partner can interact with avidin or streptavidin previously conjugated to the inert medium.

The adsorbent matrix thus produced can be contacted with a biological fluid, or a fraction thereof, through the use of an extracorporeal circuit. The development and use of extracorporal, adsorbent matrices has been extensively reviewed (see Kessler, Blood Purification 11:150-157 (1993)).

In a further aspect, herein referred to as the "stirred reactor" (Figure 2), the biological fluid can be exposed to the binding partner such as a TNFα mutein in a mixing chamber and, thereafter, the binding partner/immune system inhibitor complex can be removed by means or methods known to those skilled in the art, including, for example, by mechanical or by chemical or biological separation methods. For example, a mechanical separation method can be used in cases where the binding partner, and therefore the binding partner/immune system inhibitor complex, represent the largest components of the treated biological fluid. In those cases, filtration can be used to retain the binding partner and immune system inhibitors associated therewith, while allowing all other components of the biological fluid to permeate through the filter and, thus, to be returned to the patient. In an example of a chemical or biological separation method, the binding partner and immune system inhibitors associated therewith can be removed from the treated biological fluid through exposure to an adsorbent matrix capable of specifically attaching to the binding partner. For example, a matrix constructed with antibodies reactive with a TNFα mutein can serve this purpose. Similarly, were biotin conjugated to the binding partner such as a TNFα mutein prior to its addition to the biological fluid, a matrix constructed with avidin or streptavidin could be used to deplete the binding partner and immune system inhibitors associated therewith from the treated fluid. It is understood that removal of the binding partner/immune system inhibitor complex, such as TNFα mutein bound to TNFR, from a biological fluid can be accomplished by separating the biological fluid and the binding partner/immune system inhibitor complex in any suitable manner. Either or both the binding partner/immune system inhibitor complex and the biological fluid can be passively or actively separated from the other. Thus, for example, removal of TNFα mutein bound to TNFR from a biological fluid can be accomplished by, for example, actively removing TNFα mutein bound to TNFR from the biological fluid or actively removing the biological fluid from the TNFα mutein bound to TNFR.

In a final step of the present methods, the treated or altered biological fluid, having a reduced amount of targeted immune system inhibitor such as soluble TNF receptor, can be returned to the patient receiving treatment along with untreated fractions of the biological fluid, if any such fractions were produced during the treatment. The altered biological fluid can be administered to the mammal by any means or methods known to those skilled in the art, including, for example, by infusion directly into the circulatory system. The altered biological fluid can be administered immediately after contact with the binding partner in a contemporaneous, extracorporeal circuit. In this circuit, the biological fluid can be (a) collected, (b) separated into cellular and acellular components, if desired, (c) exposed to the binding partner, and if needed, separated from the binding partner bound to the targeted immune system inhibitor, (d) combined with the cellular component, if needed, and (e) readministered to the patient as altered biological fluid. In a further aspect, the altered acellular biological fluid can be administered to the patient at an infusion site different from the site where the cellular component of the biological fluid is administered to the patient. The administration of the altered acellular biological fluid to the patient can be simultaneous with, precede, or follow the administration of the cellular component of the biological fluid to the patient. Alternatively, the administration of the altered biological fluid can be delayed under appropriate storage conditions readily determined by those skilled in the art.

If desirable, the entire process can be repeated. Those skilled in the art can readily determine the benefits of repeated treatment by monitoring the clinical status of the patient, and correlating that status with the concentration(s) of the targeted immune system inhibitor(s) such as soluble TNFα receptor in circulation prior to, during, and after treatment.

Further provided is an apparatus for reducing the amount of a targeted immune system inhibitor such as soluble TNF receptor in a biological fluid. The apparatus can be composed of: (a) a means for separating the biological fluid into a cellular component and an acellular component or fraction thereof; (b) an adsorbent matrix having attached thereto a TNFα mutein or a stirred reactor as described above to produce an altered acellular component or fraction thereof; and (c) a means for combining the cellular fraction with the altered acellular component or fraction thereof. The apparatus is particularly useful for whole blood as the biological fluid in which the cellular component is separated either from whole plasma or a fraction thereof.

The means for initially fractionating the biological fluid into the cellular component and the acellular component, or a fraction thereof, and for recombining the cellular component with the acellular component, or fraction thereof, after treatment are known to those skilled in the art (see Apheresis: Principles and Practice, *supra*).

An immune system inhibitor to be targeted can be sTNFRI (Seckinger et al., J. Biol. Chem. 264:11966-11973 (1989); Gatanaga et al., Proc. Natl. Acad. Sci. USA 87:8781-8784 (1990)), a naturally occurring inhibitor of the pluripotent immune system stimulator, TNF. sTNFRI is produced by proteolytic cleavage, which liberates the extra-cellular domain of the membrane tumor necrosis factor receptor type I from its transmembrane and intracellular domains (Schall et al., Cell 61:361-370 (1990); Himmler et al., DNA and Cell Biol. 9:705-715 (1990)). sTNFRI retains the ability to bind to TNF with high affinity and, thus, to inhibit the binding of TNF to the membrane receptor on cell surfaces.

The levels of sTNFRI in biological fluids are increased in a variety of conditions which are characterized by an antecedent increase in TNF. These include bacterial, viral, and parasitic infections, and cancer as described above. In each of these disease states, the presence of the offending agent stimulates TNF production which stimulates a corresponding increase in sTNFRI production. sTNFRI production is intended to reduce localized, as well as systemic, toxicity associated with elevated TNF levels and to restore immunologic homeostasis.

In tumor bearing hosts, over-production of sTNFRI may profoundly affect the course of disease, considering the critical role of TNF in a variety of anti-tumor immune responses (reviewed in, Beutler and Cerami, Ann. Rev. Immunol. 7:625-655 (1989)). TNF directly induces tumor cell death by binding to the type I membrane-associated TNF receptor. Moreover, the death of vascular endothelial cells is induced by TNF binding, destroying the circulatory network serving the tumor and further contributing to tumor cell death. Critical roles for TNF in natural killer cell- and cytotoxic T lymphocyte-mediated cytolysis also have been documented. Inhibition of any or all of these effector mechanisms by sTNFRI has the potential to dramatically enhance tumor survival.

That sTNFRI promotes tumor survival, and that its removal enhances anti-tumor immunity, has been demonstrated. In an experimental mouse tumor model, sTNFRI production was found to protect transformed cells *in vitro* from the cytotoxic effects of TNF, and from cytolysis mediated by natural killer cells and cytotoxic T lymphocytes (Selinsky et al., Immunol. 94:88-93 (1998)). In addition, the secretion of sTNFRI by transformed cells has been shown to markedly enhance their tumorigenicity and persistence *in vivo* (Selinsky and Howell, Cell. Immunol. 200:81-87 (2000)). Moreover, removal of circulating sTNFRI has been found to provide clinical benefit to cancer patients, as demonstrated by human trials of Ultrapheresis as discussed above (Lentz, *supra*). These observations affirm the importance of this molecule in tumor survival and suggest the development of methods for more specific removal of sTNFRI as promising new avenues for cancer immunotherapy.

The following examples are put forth so as to provide those of ordinary skill in the art with a complete disclosure and description of how the compounds, compositions, articles, devices and/or methods claimed herein are made and evaluated and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in C or is at ambient temperature, and pressure is at or near atmospheric..

### EXAMPLE 1

### Production, Purification, and Characterization of the Immune System Inhibitor, Human sTNFRI

The sTNFRI used in the present studies was produced recombinantly either in *E. coli* (R&D Systems; Minneapolis MN) or in eukaryotic cell culture essentially as described (see U.S. Patent No. 6,379,708).

The construction of the eukaryotic expression plasmid, the methods for transforming and selecting cultured cells, and for assaying the production of sTNFRI by the transformed cells have been described (Selinsky et al., *supra*, 1998).

sTNFRI was detected and quantified in the present studies by capture ELISA (Selinsky et al., *supra*). In addition, the biological activity of recombinant sTNFRI, that is, its ability to bind TNF, was confirmed by ELISA. Assay plates were coated with human TNF α (Chemicon; Temecula CA), blocked with bovine serum albumin, and sTNFRI, contained in culture supernatants as described above, was added. Bound sTNFRI was detected through the sequential addition of biotinylated-goat anti-human sTNFRI, alkaline phosphatase-conjugated streptavidin, and p-nitrophenylphosphate.

### EXAMPLE 2

### Production, Purification, and Characterization of TNFα Muteins

Briefly, TNFα muteins 1, 2, 3 and 4 were produced by expression of the respective cDNAs in *E*. *coli.* Genes encoding TNFα and TNFα muteins 1, 2, 3 and 4 were prepared using overlapping oligonucleotides having codons optimized for bacterial expression. Each of the coding sequences was fused in frame to that encoding the ompA leader to permit export of the recombinant polypeptides to the periplasm. Synthetic fragments were cloned into ap UC19 derivative immediately downstream of the lac Z promoter, and the resulting recombinant plasmids were introduced into *E. coli.* Recombinant bacteria were cultured to late-log, induced with isopropyl-ß-D-thiogalactopyranoside (IPTG) for three hours, and harvested by centrifugation. Periplasmic fractions were prepared and tested by ELISA using polyclonal goat anti-human TNFα capture antibodies. After the addition of the diluted periplasms, bound TNFα and TNFα muteins 1, 2, 3 and 4 were detected by sequential addition of biotinylated polyclonal goat anti-human TNFα, streptavidin-alkaline phosphatase, and para-nitrophenyl phosphate (pNPP). TNFα and each of the TNFα muteins was detectable in the respective periplasms, though the level of TNFα mutein 3 only slightly exceeded the detection limit of the assay (Figure 4).

The TNFα and TNFα mutein polypeptides 1, 2 and 4 were purified from periplasmic fractions by sequential chromatography on Q and S anion and cation exchange columns, respectively, essentially as described (Tavernier et al., J. Mol. Biol. 211:493-501 (1990)). The TNFα and TNFα mutein polypeptides were purified to >95% homogeneity as analyzed by sodium dodecyl sulfate polyacrylamide gel electrophoresis (SDS-PAGE). The gels revealed a 17 kDa band corresponding to TNFα or the muteins and a 34 kDa band, which was confirmed by Western blotting to be dimerized TNFα mutein.

The TNFα muteins were tested for their ability to bind to sTNFRI. Wells of a microtiter plate were coated with TNFα, blocked, and incubated with sTNFRI either in the presence or absence of the inhibitors, TNFα and TNFα muteins 1,2 and 4. As shown in Figure 5, TNFα muteins 1, 2 and 4 each bind to sTNFRI.

### EXAMPLE 3

### Depletion of the Immune System Inhibitor, sTNFRI, From Human Plasma Using TNFα Mutein Adsorbent Matrices

The TNFα mutein adsorbent matrices were produced and tested for their ability to deplete sTNFRI from human plasma. Briefly, purified TNFα muteins 1, 2 and 4 each was conjugated to cyanogen bromide (CNBr) Sepharose™ 4B at a density of 0.5 mg per mL of beads, and the remaining CNBr groups were quenched with ethanolamine. The resulting matrices were packed in individual column housings and washed extensively with phosphate buffered saline prior to use.

Normal human plasma was spiked (33% v/v) with culture supernatant containing recombinant human sTNFRI (see Example 1) to a final concentration of 8 nanograms per milliliter and passed through the respective columns at a flow rate of one milliliter of plasma per milliliter of resin per minute. An additional column, with no immobilized protein and quenched with ethanolamine, was included to control for non-specific depletion. One mL fractions were collected, and the relative levels of sTNFRI contained in the starting material and in the fractions were determined using a capture ELISA. To perform the capture ELISA, wells were coated with polyclonal goat anti-sTNFRI, and then were blocked with 2% BSA. Plasma samples were diluted 1:2, added to the wells, and sTNFRI therein was captured. Biotinylated polyclonal goat anti-sTNFRI was added, followed by streptavidin-alkaline phosphatase, and p-nitrophenylphosphate. Relative absorbance at 405 nm was used to estimate the depletion.

As shown in Figure 6, all three of the immobilized TNFα muteins effectively depleted sTNFRI from human plasma, and the hierarchy observed in Figure 5 again was manifested. The control matrix produced no reduction in sTNFRI levels, confirming the specificity of the depletion observed with the TNFα mutein matrices. Importantly, near quantitative depletion was achieved by TNFα muteins 1 and 4 at a flow rate that approximates that anticipated for use in a clinical setting.

### EXAMPLE 4

### High Level Production and Purification of Human TNFα Mutein 4

A high level expression system for producing human TNFα mutein 4 was developed. A synthetic gene encoding human TNFα mutein 4 was produced by Blue Heron Biotechnology (Bothell, WA) from a series of overlapping oligonucleotides. The 5' end of the gene contains a site for the restriction endonuclease *Nde I*, which includes a methionine codon within the hexanucleotide recognition sequence. This methionine codon is fused in-frame to codons for six histidine residues (⁶His), and these are followed by additional codons that specify the seven amino acid recognition sequence cleaved by tobacco etch virus protease (TEV). This recognition sequence is followed by a sequence encoding 157 amino acids that represent the entire extracellular portion of the native TNFα polypeptide, except for the substitution of tyrosine for serine at position 147. An in-frame termination codon follows the TNFα mutein coding sequence and the termination codon is followed by a site for the restriction endonuclease *Bam HI.* Except for the restriction endonuclease sites, the construct was optimized to reflect the codon bias of *E. coli* and to minimize secondary structure of the mRNA transcript. The synthetic gene was cloned into a derivative of pUC19 that is proprietary to Blue Heron, and the nucleotide sequence of the coding region was confirmed on both strands using the dideoxy chain termination method.

The prokaryotic expression plasmid, pET-11a (EMD Biosciences, San Diego, CA), was used to construct the high-level expression vector. The synthetic gene encoding ⁶His-TEV-TNFα Mutein 4 was liberated from the pUC19-based recombinant plasmid described above by digestion with *Nde I* and *Bam HI.* The resulting fragment was purified by agarose gel electrophoresis and ligated into unique *Nde I* and *Bam HI* sites in pest-11a. Products of the ligation reaction were introduced by electroporation into the *E. coli* host strain, λBL21 (DE3) (EMD Biosciences). λBL21 (DE3) is a derivative of *E. coli* K 12, which contains a lysogenic λ phage integration that has transduced the RNA polymerase of T7 bacteriophage into the bacterial genome. Inducible expression of the T7 polymerase gene allows high-level transcription from the T7 promoter present immediately upstream of the TNFα mutein gene in pET-11a. Stably transformed bacteria were selected on Luria Bertani plates containing 100 ug/mL ampicillin. Individual colonies were selected based on the fidelity of the expression plasmid as assessed through the isolation of the recombinant plasmids and the liberation of the ⁶His-TEV-TNFα Mutein 4 gene by combined digestion with NdeI and *Bam HI.*

Cultures of λBL21 (DE3) transformed with the TNFα mutein 4 expression construct were grown at 37° C, induced with IPTG, and harvested by centrifugation. Bacterial pellets were resuspended in lysis buffer, and incubated for 30 minutes with DNAse and lysozyme. The lysate was homogenized and clarified by centrifugation. TNFα mutein 4 contained within the lysate were purified using His Bind column (EMD Biosciences) according to the manufacturer's instructions. Purified TNFα mutein 4 was digested with TEV protease and the reaction mixture was rechromatographed on a His Bind column to remove the dissociated polyhistidine tag and the TEV protease, which also contains a polyhistidine tag. The purified TNFα mutein 4 was collected in the His Bind column flow through, dialyzed against phosphate buffered saline, and stored at -20°C. The purity of the preparation was >95%, as assessed by sodium dodecyl sulfate polyacrylamide gel electrophoresis (SDS-PAGE).

### EXAMPLE 5

### In Vitro Depletion of sTNFRI and sTNFRII From Plasma Using an Extracorporeal Adsorbent Device Constructed with TNFα Mutein 4

An extracorporeal adsorbent device was produced sterilely though the chemical conjugation of purified TNFα mutein 4 to Actigel ALD Ultraflow 4 (Sterogene Bioseparations, Carlsbad, CA). Actigel ALD Ultraflow is preferred for construction of the adsorbent device due to the superior stability of the aldehyde chemistry used for ligand conjugation, and the proven biocompatibility and stability of cross-linked agarose beads in blood and plasma. TNFα mutein 4 was conjugated to the beads at a density of 1 mg of mutein per mL of beads according to the manufacturer's instructions. Sixty-two and one-half mL of the matrix was packed by gravity flow into a polycarbonate housing (5 cm long x 4 cm diameter), which contains 36 micron polypropylene filters at either end. The resulting device was washed sequentially with ten column volumes of 0.5 M NaCl, ten column volumes of 0.1 M glycine, pH 2.8, and ten column volumes of normal saline containing 0.1% sodium azide.

The TNFα mutein 4 adsorbent device was evaluated *in vitro* for its ability to deplete human sTNFR from plasma. Normal human plasma was filtered through a 0.45 micron filter and pumped through the device at a flow rate of thirty milliliters of plasma per minute. Thirty twenty-five mL fractions were collected, and the levels of human sTNFRI and sTNFRII contained in the initial plasma sample and in the fractions were determined using sTNFRI and sTNFRII capture ELISAs. To perform the capture ELISAs, wells were coated with either polyclonal goat anti-human sTNFRI or polyclonal goat anti-human sTNFRII (R&D Systems), and then were blocked with 2% BSA. Plasma samples were diluted 1:2, added to the wells, and sTNFRI or sTNFRII therein was captured. Biotinylated polyclonal goat anti-human sTNFRI or biotinylated polyclonal goat anti-human sTNFRII (R&D Systems) were added, followed by streptavidin-alkaline phosphatase, and p-nitrophenylphosphate. The absorbance at 405 nm for each sample was used to calculate the concentration of sTNFRI and sTNFRII relative to standard curves obtained using purified human sTNFRI and sTNFRII (R&D Systems). The concentrations of human sTNFRI and sTNFRII in the initial plasma sample were 1.17 ng/mL and 0.55 ng/mL, respectively. sTNFRI and sTNFRII were not detectable in any of the fractions collected after passage through the device, confirming the ability of the device to deplete both of these low abundance solutes from plasma.

The TNFα mutein 4 adsorbent device was evaluated in an identical manner for its ability to deplete canine sTNFR from plasma. Normal human plasma, previously depleted of endogenous human sTNFRI and sTNFRII by passage through the TNFα mutein 4 adsorbent device, was spiked with canine sTNFRI and sTNFRII at 2 ng/mL and 5 ng/mL, respectively. The canine sTNFR used in this analysis were produced recombinantly in cell culture as described in Example 7 below. The spiked plasma was filtered through a 0.45 micron filter and pumped through the device at a flow rate of thirty milliliters of plasma per minute. Twenty-five ten mL fractions were collected, and the levels of canine sTNFRI and sTNFRII contained in the initial plasma sample and in the fractions were determined using sTNFRI and sTNFRII capture ELISAs. To perform the capture ELISAs, wells were coated with either polyclonal goat anti-human sTNFRI or polyclonal goat anti-human sTNFRII as above. After blocking with 2% BSA, plasma samples were diluted 1:2 and added to the wells, and the respective sTNFR therein were captured. Polyclonal rabbit IgG, reactive with the carboxy terminal polyhistidine tag of recombinant canine sTNFRI and sTNFRII , was added, followed by biotinylated-anti-rabbit IgG, streptavidin-allcaline phosphatase, and p-nitrophenylphosphate. The absorbance at 405 nm for each sample was used to calculate the concentration of sTNFRI and sTNFRII relative to standard curves obtained using purified recombinant canine sTNFRI and sTNFRII. The concentrations of canine sTNFRI and sTNFRII in the spiked plasma sample, as assessed by ELISA, were determined to be 2.8 ng/mL and 4.0 ng/mL, respectively. After passage through the adsorbent device, sTNFRI concentrations in the plasma fractions were reduced from 57% to 82%, and sTNFRII was undetectable in any of the plasma fractions. These findings confirm the ability of the human TNFα mutein 4 adsorbent device to deplete canine sTNFR as well.

### EXAMPLE 6

### Ex Vivo Depletion of Canine sTNFRI From Plasma Using an Extracorporeal Adsorbent Device Constructed with TNFα Mutein 4

The ability of the TNFα mutein 4 adsorbent device to deplete sTNFRI from plasma, when used in an extracorporeal circuit not unlike that employed for therapeutic plasma exchange, was evaluated. Briefly, blood was removed from a 47 kg canine through a dual-lumen catheter implanted in the external jugular. The blood was delivered to Cobe Spectra, a centrifugal plasma separator, using a Cobe Spectra Therapeutic Plasma Exchange disposable tubing set that previously had been modified sterilely by CytoLogic. Once separated, the blood cells and plasma were routed, independent of each other, through the remainder of the system. The plasma component was passed through the TNFα mutein 4 adsorbent device as illustrated in Figure 1 herein. The treated plasma was recombined with the blood cells and returned through the catheter to the animal. Several procedures, in which one-half to one plasma volume was treated, were performed and each was completed in less than one hour.

Depletion of canine sTNFRI by this process was evaluated. Plasma samples were obtained from the extracorporeal circuit immediately prior to entering the adsorbent device and immediately after exiting the adsorbent device. The levels of canine sTNFRI in these plasma samples were determined using a capture ELISA. To perform the capture ELISA, wells were coated with monoclonal mouse anti-human sTNFRI (Becton-Diclcinson, Mountain View, CA), previously demonstrated to cross react with canine sTNFRI. Wells then were blocked with 2% BSA. Pre- and post device plasma samples were diluted 1:2, added to the wells, and the canine sTNFRI therein was captured. Biotinylated polyclonal goat anti-human sTNFRI was added, followed by streptavidin-alkaline phosphatase, and p-nitrophenylphosphate. The absorbance at 405 nm for each sample was used to calculate the concentration of sTNFRI relative to a standard curve obtained using purified recombinant canine sTNFRI. Pairs of pre- and post-device plasma samples, obtained during five separate treatments of this subject, were evaluated using this assay (see table below). In each of the sample pairs, significant depletion of sTNFRI was observed (range = 69.70% to 81.03 percent). The mean percent depletion of sTNFRI for these five treatments was 74.90 ± 5.66.

| **Treatment** | **Pre-Device Plasma sTNFRI (ng/mL)** | **Post-Device Plasma sTNFRI (ng/mL)** | **Percent Depletion*** |
|---|---|---|---|
| Day 1 | 1.65 | 0.50 | 69.70 |
| Day 8 | 0.40 | 0.12 | 70.00 |
| Day 16 | 0.68 | 0.13 | 80.88 |
| Day 19 | 0.58 | 0.11 | 81.03 |
| Day 23 | 0.48 | 0.13 | 72.91 |

| | | | |
|---|---|---|---|
| • Mean Depletion = 74.90 ± 5.66 | | | |

### EXAMPLE 7

### Production and Purification of Canine sTNFRI and sTNFRII

Canine sTNFR used in these studies were produced in eukaryotic cell culture. Synthetic genes encoding canine sTNFRI and sTNFRII were produced by Blue Heron Biotechnology from a series of overlapping oligonucleotides. The sequence of the canine sTNFRI cDNA was based on that contained in Genbank Accession number XM_849381. The sequence of the canine sTNFRII cDNA was based on that contained in Genbank Accession number XM-544562. Each of the cDNAs were fused in-frame at the 3' end to codons for six histidine residues (⁶His) followed by an in-frame termination codon. The 5' and 3' ends of each gene were flanked by recognition sequences for the restriction endonucleases *Hind III* and *Xho I,* respectively. Except for the restriction endonuclease sites, each construct was optimized to reflect the codon bias of *Canis familiaris* and to minimize secondary structure of the mRNA transcript. Each synthetic gene was cloned into a derivative of pUC19 that is proprietary to Blue Heron, and the nucleotide sequence of each coding region was confirmed on both strands using the dideoxy chain termination method.

The synthetic genes encoding canine sTNFRI and sTNFRII were liberated from the pUC19-based recombinant plasmids described above by digestion with *Hind III* and *Xho I.* The resulting fragments were purified by agarose gel electrophoresis and ligated into unique *Hind III* and *Xho I* sites in the eukaryotic expression vector, pCEP4 (Invitrogen, Carlsbad, CA). Products of the ligation reactions were introduced by electroporation into the *E. coli* host strain, λBL21 (EMD Biosciences). Stably transformed bacteria were selected on Luria Bertani plates containing 100 ug/mL ampicillin. Individual colonies were selected based on the fidelity of the expression plasmid as assessed through the isolation of the recombinant plasmids and the liberation of the respective sTNFR genes by combined digestion with *Hind III* and *Xho I.*

The canine sTNFR expression plasmids were introduced into cultures of Cf2Th cells (American Type Culture Collection, Manassas, VA) using lipofectamine. Cloned transfectant cell lines producing sTNFRI (Clone E) and sTNFRII (Clone F) were isolated by limiting dilution in the presence of hygromycin (0.1 mg active drug/ml). Cultures of Clone E and Clone F were grown in spinner flasks, and recombinant canine sTNFRI and sTNFRII, respectively, were purified by affinity chromatography on columns of human TNFα or human TNFR mutein 4 immobilized on cyanogen bromide Sepharose. Purified sTNFR were dialyzed against PBS and stored at -20°C.

## Claims

1. An adsorbent matrix that facilitates the depletion of a targeted soluble TNF receptor from a mammalian biological fluid in an extracorporeal system, the adsorbent matrix comprising:
a plurality of tumor necrosis factor-alpha (TNF-alpha) muteins immobilized on a solid support that is part of the adsorbent matrix and is configured to remain in the extracorporeal system, wherein the plurality of TNF-alpha muteins is selected from mutein 1, which is identified by SEQ ID NO:3; mutein 2, which is identified by SEQ ID NO:4; and mutein 4, which is identified by SEQ ID NO:6.

2. The adsorbent matrix of claim 1, wherein the plurality of TNF-alpha muteins is covalently attached to the solid support.

3. The adsorbent matrix of claim 1, wherein the solid support is an inert medium.

4. The adsorbent matrix of claim 1, wherein the solid support is in the form of a bead.

5. The adsorbent matrix of claim 4, wherein the bead is a macroporous bead.

6. The adsorbent matrix of claim 5, wherein the macroporous bead is selected from agarose, cross-linked agarose, cellulose, controlled pore glass, polyacrylamide, azlactone, polymethacrylate, and polystyrene.

7. The adsorbent matrix of claim 1, wherein the plurality of tumor necrosis factor-alpha (TNF-alpha) muteins immobilized on the solid support is contained within a mixing chamber of the extracorporeal system.

## Patentansprüche

1. Adsorbierende Matrix, die die Depletion eines angezielten löslichen TNF-Rezeptors aus einer biologischen Flüssigkeit eines Säugetiers in einem extrakorporalen System erleichtert, wobei die adsorbierende Matrix
eine Vielzahl von Tumornekrosefaktor-alpha(TNF-alpha)-Muteinen aufweist, die auf einem festen Träger immobilisiert sind, der Teil der adsorbierenden Matrix ist und so konfiguriert ist, dass er in dem extrakorporalen System verbleibt, wobei die Vielzahl von TNF-alpha-Muteinen aus Mutein 1, das durch SEQ ID NO:3 identifiziert ist; Mutein 2, das durch SEQ ID NO:4 identifiziert ist; und Mutein 4, das durch SEQ ID NO: 6 identifiziert ist, ausgewählt ist.

2. Adsorbierende Matrix nach Anspruch 1, wobei die Vielzahl von TNF-alpha-Muteinen kovalent an dem festen Träger befestigt ist.

3. Adsorbierende Matrix nach Anspruch 1, wobei der feste Träger ein inertes Medium ist.

4. Adsorbierende Matrix nach Anspruch 1, wobei der feste Träger in Form eines Kügelchens vorliegt.

5. Adsorbierende Matrix nach Anspruch 4, wobei das Kügelchen ein makroporöses Kügelchen ist.

6. Adsorbierende Matrix nach Anspruch 5, wobei das makroporöse Kügelchen aus Agarose, vernetzter Agarose, Cellulose, kontrolliert porösem Glas, Polyacrylamid, Azlacton, Polymethacrylat, und Polystyrol ausgewählt ist.

7. Adsorbierende Matrix nach Anspruch 1, wobei die Vielzahl von auf dem festen Träger immobilisierten Tumornekrosefaktor-alpha(TNF-alpha)-Muteinen in einer Mischkammer des extrakorporalen Systems enthalten ist.

## Revendications

1. Matrice adsorbante qui facilite la déplétion d'un récepteur de facteur nécrosant des tumeurs (TNF) soluble cible d'un fluide biologique de mammifère dans un système extracorporel, la matrice adsorbante comprenant :
une pluralité de mutéines de facteur nécrosant des tumeurs alpha (TNF-alpha) immobilisées sur un support solide qui fait partie de la matrice adsorbante et est configuré pour rester dans le système extracorporel, où la pluralité de mutéines de TNF-alpha est sélectionnée parmi la mutéine 1, laquelle est identifiée par la séquence à numéro d'identification SEQ ID N° 3 ; la mutéine 2, laquelle est identifiée par la séquence à numéro d'identification SEQ ID N° 4 ; et la mutéine 4, laquelle est identifiée par la séquence à numéro d'identification SEQ ID N° 6.

2. Matrice adsorbante selon la revendication 1, dans laquelle la pluralité de mutéines de TNF-alpha est liée de manière covalente au support solide.

3. Matrice adsorbante selon la revendication 1, dans laquelle le support solide est un milieu inerte.

4. Matrice adsorbante selon la revendication 1, dans laquelle le support solide se présente sous la forme d'une bille.

5. Matrice adsorbante selon la revendication 4, dans laquelle la bille est une bille macroporeuse.

6. Matrice adsorbante selon la revendication 5, dans laquelle la bille macroporeuse est sélectionnée parmi de l'agarose, de l'agarose réticulée, de la cellulose, du verre à porosité contrôlée, du polyacrylamide, de l'azlactone, du polyméthacrylate, et du polystyrène.

7. Matrice adsorbante selon la revendication 1, dans laquelle la pluralité de mutéines de facteur nécrosant des tumeurs alpha (TNF-alpha) immobilisées sur le support solide est contenue dans une chambre de mélange du système extracorporel.
